# EUROPEAN PATENT SPECIFICATION

(11) **EP 1 926 462 B1**
(45) Date of publication and mention of the grant of the patent: **20.10.2010**
(21) Application number: 06795953.6
(22) Date of filing: 07.09.2006
(51) Int. Cl.: A61F 13/64

(54) **DISPOSABLE ABSORBENT ARTICLE HAVING DEPLOYABLE BELT STRIPS**
SAUGFÄHIGER EINWEGARTIKEL MIT ENTFALTBAREM GURTBAND
ARTICLE ABSORBANT JETABLE POURVU DE BANDES FORMANT CEINTURE DEPLOYABLES

(30) Priority: 21.09.2005 US 231500
(43) Date of publication of application: 04.06.2008
(73) Proprietor: The Procter and Gamble Company, Cincinnati, Ohio 45202 (US)
(72) Inventor: LAVON, Gary, Dean, Hamilton, OH 45011 (US); HAMALL, Kenneth, Michael, West Chester, OH 45069 (US); BECK, Theodora, Cincinnati, OH 45251 (US); HAYDEN, Michael, Patrick, Mason, OH 45040 (US); LUDWIG, Susan, Joy, West Chester, OH 45069 (US)
(74) Representative: Heide, Ute
(86) International application number: PCT/IB2006/053161
(87) International publication number: WO 2007/034349

(56) References cited:
- EP-A1- 0 460 467
- EP-A2- 0 287 388
- WO-A-2005/016211
- WO-A-2005/074851
- ES-A1- 2 213 491
- FR-A1- 2 566 631
- GB-A- 2 242 612
- US-A- 3 386 442
- US-A- 6 110 157

## Description

### FIELD OF THE INVENTION

This invention relates to disposable absorbent articles such as disposable diapers and other articles intended for use on incontinent persons.

### BACKGROUND OF THE INVENTION

Disposable absorbent articles are designed to absorb and contain bodily waste in order to prevent soiling of the body and clothing of the wearer, as well as bedding or other objects with which the wearer comes into contact.

As the usage of disposable absorbent articles has expanded, their complexity has increased with the incorporation of additional features serving to enhance their performance and appearance. Among these are often complex waist closure components for application onto the body of a wearer. The costs of the materials and the costs of the manufacturing processes have also increased in conjunction with the increased in complexity. As a result, the prices at which these articles are sold have risen to levels that many potential purchasers around the world cannot afford to pay. Thus, a need exists for a disposable absorbent article having simple and cost-effective waist closure means.

EP 0 287 388 discloses an integral disposable absorbent garment, such as a disposable diaper or incontinent brief, having an integral belt segment on each side of the garment. The integral belt segments, in association with a receiving or attachment means, are intended to hold or suspend the garment about the lower torso of the wearer.

US 3 386 442 discloses a disposable diaper made from stock of generally rectangular over-all outline which has its intermediate portion of materially less width than end portions of the diaper, a moisture absorbent filler which is centered on an impermeable backing and anchored by a moisture permeable facing sheet which is fastened to the moistureproof backing beyond the filler. Side strips connected with the backing sheet beyond the filler are also connected with the facing sheet over the filler, thereby sealing and strengthening the side margins.

US 6 110 157 discloses a disposable absorbent article which has an integrated fastening system. The integrated fastening system comprises a pair of belt segments which are provided by and at least partially detachable from the longitudinal end portions or side portions of the absorbent article. Each of the belt segments includes a free end which is configured to be detachable from the absorbent article. The integrated fastening system also comprises a fastening means for attaching the free end of each of the belt segments to the absorbent article to maintain the absorbent article about a waist of a wearer when in use. A reinforcement panel may also be located on the portion of the absorbent article used to provide the belt segments to reinforce the belt segments.

FR 2 566 631 discloses nappy-pants comprising first elastic means fastened in the stretched state along two longitudinal edges of the absorbent pad and second elastic means fastened in the stretched state on two waistband strips which, before use, are joined by a tearable, longitudinal weakening line to the remaining part of the nappy-pants.

WO 2005/074851 discloses a simple disposable absorbent article including a chassis and an absorbent assembly. The chassis includes a water-impermeable sheet folded laterally inward at both of its side edges to form opposing side flaps. Each side flap is attached to the interior surface of the chassis adjacent to its end edges. Each side flap has a longitudinally extending elastic gathering member attached adjacent to its proximal edge. The absorbent assembly is smaller in width and in length than the chassis. The side edges and end edges of the absorbent assembly may be disposed proximally relative to the respective side edges and end edges of the chassis. The absorbent assembly includes an absorbent core that may contain superabsorbent particles, which may be contained inside pockets. The chassis may be extensible. The absorbent assembly may be attached in a cruciform pattern to the chassis to allow portions of the chassis to extend laterally.

FR 2 612 770 discloses an incontinence pad. The pad is formed on the one hand by a towel, formed by a leaktight outer sheet, an absorbent wad and a permeable inner sheet, and whose longitudinal direction has an outline adapted to pass through the crotch of the incontinent person, and on the other hand by means holding the towel at the waist of the patient. According to the invention, the holding means are formed by two belt elements which are fixed at rest in the longitudinal direction of the towel, and which, when used, fold transversely to the towel so as to allow them to pass around the waist of the patient.

US H1440 discloses a disposable absorbent garment having a belt. The belt may either be integral with or detachable from the absorbent portion of the garment. The belt is arcuate so that one laterally extending edge of the belt is longer than the other laterally extending edge of the belt. This arrangement provides a better fitting belt which more discretely conforms to the contours of the wearer and is less noticeable under clothing.

### SUMMARY OF THE INVENTION

The present invention is a disposable absorbent article having laterally opposing interiorly attached side flaps and at least one deployable belt strip. Each side flap is formed by folding a portion of the absorbent assembly laterally inward and has a longitudinally extending elastic gathering member attached adjacent to its proximal edge. The belt strip has a fixed end portion and opposing first and second edges connecting the fixed end portion and an opposing free end portion. The belt strip is attached in the fixed end portion and is deployed by being folded laterally outward such that the first edge extends laterally outward from one end point of a diagonal fold line and the second edge extends laterally outward from the opposing end point of the diagonal fold line. The belt strip may be tied to another belt strip or may be fastened to the waist region of the article or to another belt strip.

### BRIEF DESCRIPTION OF THE DRAWINGS

In the accompanying drawing figures, like reference numerals identify structurally corresponding elements, which may or may not be identical in the several exemplary embodiments that are depicted. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description.

In the drawing figures and in the written description, lowercase letters appended to reference numerals indicate generally symmetric elements, *e.g*., left and right symmetric elements may be respectively identified by the reference numerals **1a** and **1b.** A preference numeral without an appended lowercase letter identifies all of the elements to which that particular reference numeral applies, *e.g*., the same elements as a group may be designated 1.

Figurs 43 to 48, Figures 54 to 56 and Figures 65 to 67 are not considered as showing embodiments of the present invention
**Figure 1** is a plan view of an exemplary disposable absorbent article in the form of a disposable diaper **20** in which the interior portion of the diaper is shown facing the viewer.
**Figure 2** is another interior plan view of the diaper **20** of **Figure 1** in which the belt strips **500** have been deployed by being folded laterally outward.
**Figure 3** is a perspective view of the diaper **20** of **Figure 2****.**
**Figure 4, Figure 5****,** and **Figure 6** are respectively simplified side, front, and back elevation views of the diaper **20** of **Figure 1** being worn about the lower torso of a wearer.
**Figure 7, Figure 8,** and **Figure 9** are plan views of portions of exemplary diapers **20** showing alternative attachment patterns **508.**
**Figure 10** is an exterior plan view of another exemplary disposable diaper **20.** **Figure 11** is another exterior plan view of the diaper **20** of **Figure 10** in which the belt strips **500** have been deployed by being folded laterally outward.
**Figure 12** is a perspective view of the diaper **20** of **Figure 11****.**
**Figure 13, Figure 14****,** and **Figure 15** are respectively simplified side, front, and back elevation views of the diaper **20** of **Figure 10** being worn about the lower torso of a wearer.
**Figure 16, Figure 17,** and **Figure 18** are plan views of portions of exemplary diapers **20.**
**Figure 19** is an exterior plan view of another exemplary disposable diaper **20.**
**Figure 20** is an exterior plan view of another exemplary disposable diaper **20.**
**Figure 21** is a perspective view of the diaper **20** of **Figure 20** showing the belt strips **500** deployed.
**Figure 22** and **Figure 23** are respectively simplified side and front elevation views of the diaper **20** of **Figure 20** being worn about the lower torso of a wearer.
**Figure 24** is an exterior plan view of another exemplary disposable diaper **20.**
**Figure 25** is a perspective view of the diaper **20** of **Figure 24** showing the belt strips **500** deployed.
**Figure 26** and **Figure 27** are respectively simplified side and front elevation views of the diaper **20** of **Figure 24** being worn about the lower torso of a wearer.
**Figure 28** is an exterior plan view of another exemplary disposable diaper **20.**
**Figure 29** is a perspective view of the diaper **20** of **Figure 28** showing the belt strips **500** deployed.
**Figure 30** is a simplified side elevation view of the diaper **20** of **Figure 28** being worn about the lower torso of a wearer.
**Figure 31** is an exterior plan view of another exemplary disposable diaper **20.**
**Figure 32, Figure 33****,** and **Figure 34** are respectively simplified side, front, and back elevation views of the diaper **20** of **Figure 31** being worn about the lower torso of a wearer.
**Figure 35** is an exterior plan view of another exemplary disposable diaper **20.**
**Figure 36** is a perspective view of an alternative embodiment of a diaper **20.**
**Figure 37** is a perspective view of another alternative embodiment of a diaper **20.**
**Figure 38** is an interior plan view of another exemplary disposable diaper **20.** **Figure 39** is an exterior plan view of the disposable diaper **20** of **Figure 38****.**
**Figure 40, Figure 41,** and **Figure 42** are section views of the diaper **20** of **Figure 38** and **Figure 39** taken at the respective section lines **40-40, 41-41,** and **42-42.** In these section views, the interior portion of the diaper **20** is shown facing upward.
**Figure 43, Figure 44,** and **Figure 45** are section views of an alternative embodiment of a diaper **20** taken at section lines corresponding to the respective section lines **40-40, 41-41,** and **42-42.** In these section views, the interior portion of the diaper **20** is shown facing upward.
   **Figure 46, Figure 47,** and **Figure 48** are section views of an alternative embodiment of a diaper **20** taken at section lines corresponding to the respective section lines **40-40, 41-41,** and **42-42.** In these section views, the interior portion of the diaper **20** is shown facing upward.
   **Figure 49** is an interior plan view of another exemplary disposable diaper **20.** **Figure 50** is an exterior plan view of the disposable diaper **20** of **Figure 49****.**
   **Figure 51, Figure 52,** and **Figure 53** are section views of the diaper **20** of **Figure 49** and **Figure 50** taken at the respective section lines **51-51, 52-52,** and **53-53.** In these section views, the interior portion of the diaper **20** is shown facing upward.
   **Figure 54, Figure 55,** and **Figure 56** are section views of an alternative embodiment of a diaper **20** taken at section lines corresponding to the respective section lines **51-51, 52-52,** and **53-53.** In these section views, the interior portion of the diaper **20** is shown facing upward.
   **Figure 57, Figure 58,** and **Figure 59** are section views of an alternative embodiment of a diaper **20** taken at section lines corresponding to the respective section lines **51-51, 52-52,** and **53-53.** In these section views, the interior portion of the diaper **20** is shown facing upward.
   **Figure 60** is an interior plan view of another exemplary disposable diaper **20.** **Figure 61** is an exterior plan view of the disposable diaper **20** of **Figure 60****.**
   **Figure 62, Figure 63,** and **Figure** 64 are section views of the diaper **20** of **Figure 60** and **Figure 61** taken at the respective section lines **62-62, 63-63,** and **64-64.** In these section views, the interior portion of the diaper **20** is shown facing upward.
   **Figure 65, Figure 66,** and **Figure 67** are section views of an alternative embodiment of a diaper **20** taken at section lines corresponding to the respective section lines **62-62, 63-63,** and **64-64.** In these section views, the interior portion of the diaper **20** is shown facing upward.
   **Figure 68, Figure 69,** and **Figure 70** are section views of an alternative embodiment of a diaper **20** taken at section lines corresponding to the respective section lines **62-62, 63-63,** and **64-64.** In these section views, the interior portion of the diaper **20** is shown facing upward.
**Figure 71, Figure 72,** and **Figure 73** are section views of an alternative embodiment of a diaper **20** taken at section lines corresponding to the respective section lines **62-62, 63-63,** and **64-64.** In these section views, the interior portion of the diaper **20** is shown facing upward.

### DETAILED DESCRIPTION OF THE INVENTION

In this description, the following terms have the following meanings:

The term "absorbent article" refers to a device that absorbs and contains liquid, and more specifically, refers to a device that is placed against or in proximity to the body of a wearer to absorb and contain the various exudates discharged from the body.

The term "diaper" refers to an absorbent article that is generally worn by infants and incontinent persons about the lower torso so as to encircle the waist and the legs of the wearer and that is specifically adapted to receive and contain urinary and fecal waste.

The term "disposable" refers to the nature of absorbent articles that generally are not intended to be laundered or otherwise restored or reused as an absorbent article, *i.e.,* they are intended to be discarded after a single use and, preferably, to be recycled, composted or otherwise disposed of in an environmentally compatible manner. In this description, a disposable diaper is described as being representative of an exemplary disposable absorbent article.

The term "deploy" in all its forms refers to the manipulation of the disclosed belt strips from their initial configuration to a configuration in which they can be used to at least partially encircle the waist of a wearer of the article on which they are provided.

The term "longitudinal" refers to a direction running from a waist edge to an opposing waist edge of the article and generally parallel to the maximum linear dimension of the article.

The term "lateral" refers to a direction running from a side edge to an opposing side edge of the article and generally at a right angle to the longitudinal direction.

The term "diagonal" refers to an orientation of a line extending obliquely relative to the longitudinal and lateral directions, *i.e*., neither perpendicular nor parallel to either of the longitudinal or lateral directions.

The term "disposed" refers to an element being attached and positioned in a particular place or position in a unitary structure with other elements.

The term "attached" refers to elements being connected or united by fastening, adhering, bonding, *etc.* by any method suitable for the elements being attached together and their constituent materials. Many suitable methods for attaching elements together are well-known, including adhesive bonding, pressure bonding, thermal bonding, mechanical fastening, *etc.* Such attachment methods may be used to attach elements together over a particular area either continuously or intermittently.

The term "cohesive" refers to the property of a material that, once set, sticks to itself but does not to any significant degree stick to other materials.

The terms "water-permeable" and "water-impermeable" refer to the penetrability of materials in the context of the intended usage of disposable absorbent articles. Specifically, the term "water-permeable" refers to a layer or a layered structure having pores, openings, and/or interconnnected void spaces that permit liquid water to pass through its thickness in the absence of a forcing pressure. Conversely, the term "water-impermeable" refers to a layer or a layered structure through the thickness of which liquid water cannot pass in the absence of a forcing pressure. A layer or a layered structure that is water-impermeable according to this definition may be permeable to water vapor, *i.e*., may be "water vapor-permeable". Such a water vapor-permeable layer or layered structure is commonly known in the art as "breathable". As is well known in the art, a common method for measuring the permeability to water of the materials typically used in absorbent articles is a hydrostatic pressure test, also called a hydrostatic head test or simply a "hydrohead" test. Suitable well known compendial methods for hydrohead testing are approved by INDA (formerly the International Nonwovens and Disposables Association, now The Association of the Nonwoven Fabrics Industry) and EDANA (European Disposables And Nonwovens Association).

The terms "proximal" and "distal" refer respectively to the location of an element relatively near to or far from the center of a structure, *e.g*., the laterally proximal edge of a longitudinally extending element is located nearer to the longitudinal axis than the laterally distal edge of the same element is located relative to the same longitudinal axis. When used to describe relative locations with respect to the axes, synonyms include "inboard" and "outboard", respectively.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside". Also, when the absorbent article is oriented such that its interior faces upward, *e.g*., when it is laid out in preparation for setting the wearer on top of it, synonyms include "upper" and "lower", "above" and "below", "over" and "under", and "top" and "bottom", respectively.

As can be seen in the drawing figures, one end portion of the exemplary diaper **20** is configured as a front waist region **36,** the longitudinally opposing end portion is configured as a back waist region **38,** and an intermediate portion is configured as a crotch region **37.**

The basic structure of the diaper **20** includes a chassis **100,** which has a front edge **136,** a back edge **138,** laterally opposing side edges **137,** an interior surface **102,** and an exterior surface **104.** A longitudinal axis **42** extends through the midpoints of the front edge **136** and the back edge **138** and a lateral axis **44** extends through the midpoints of the side edges **137.**

The basic structure of the diaper **20** also includes an absorbent assembly **200,** which is attached to the chassis **100.** The absorbent assembly **200** absorbs and retains liquid bodily waste materials. Suitable well-known absorbent materials for the absorbent assembly include cellulose fibers in the form of comminuted wood pulp, which is commonly known as "airfelt", layers or sheets of natural or synthetic fibrous material, superabsorbent polymer, *etc.* These absorbent materials may be used separately or in combination and many may be used in a discrete form, *i.e.,* in the form of fibers, granules, particles, layers and the like. The discrete form of an absorbent material may be immobilized in pockets formed by a layer of a thermoplastic material, such as a hot melt adhesive, that intermittently contacts and adheres to a substrate, such as a covering sheet, while diverging away from the substrate at the pockets. Absorbent assemblies having such pocket structures are described in more detail in U.S. Patent Application Publications Nos. 2004/0167486 of 26 August 2004 and 2004/0162536 of 19 August 2004.

The basic structure of the diaper **20** also includes at least one deployable belt strip **500**, as described in detail below.

When the diaper **20** is worn on the lower torso of a wearer, the front waist edge **136** and the back waist edge **138** of the chassis lie against the waist of the wearer, the side edges **137** partially or wholly encircle the legs of the wearer, the crotch region **37** is generally positioned between the legs of the wearer, and the absorbent assembly **200** extends from the front waist region **36** through the crotch region **37** to the back waist region **38.**

A portion or the whole of chassis and/or the absorbent assembly and/or the belt strip may be formed of an elastically extensible material or materials. Alternatively, or in addition, a portion or the whole of chassis and/or the absorbent assembly and/or the belt strip may be made extensible to a degree greater than the inherent extensibility of the material or materials from which it is made. The additional extensibility may be desirable in order to allow the diaper **20** to conform to the body of a wearer during movement by the wearer. Additional lateral extensibility may be particularly desirable to allow the user of a diaper to extend the front waist region and/or the back waist region to encircle the waist of a wearer, *i.e*., to tailor the waist size and fit of a diaper to the individual wearer. Such a lateral extension of the waist region or regions may give the diaper a generally hourglass shape and may impart a tailored appearance to the diaper when it is worn. In addition, the additional extensibility may be desirable in order to minimize the cost of the diaper, because a relatively lesser amount of material is needed when the material is made extensible as described.

This additional extensibility may be provided in a variety of ways. For example, a material or materials from which the chassis and/or the absorbent assembly and/or the belt strip is/are made may be pleated by any of many known methods. Alternatively, all or a portion of the chassis and/or the absorbent assembly and/or the belt strip may be made of a formed web material or a formed laminate of web materials like those described in U.S. Patent No. 5,518,801 issued on 21 May 1996 in the name of Chappell et al*.* In addition, different portions of the chassis and/or the absorbent assembly and/or the belt strip may be formed to have different ranges of extensibility and/or to be extensible to a greater or lesser degree when subjected to a given level of opposing tensile forces, *i.e.,* to be relatively more easily or less easily extensible. Such differential extensibility may be desirable so that, for example, one or both of the waist regions may be laterally extended relatively farther or relatively more easily than the crotch region.

Unless explicitly excluded in its description or precluded by a structural characteristic unique to the particular disposition of the belt strip **500** or to the particular embodiment shown, the following description of alternatives applies to every configuration of the belt strip **500.**

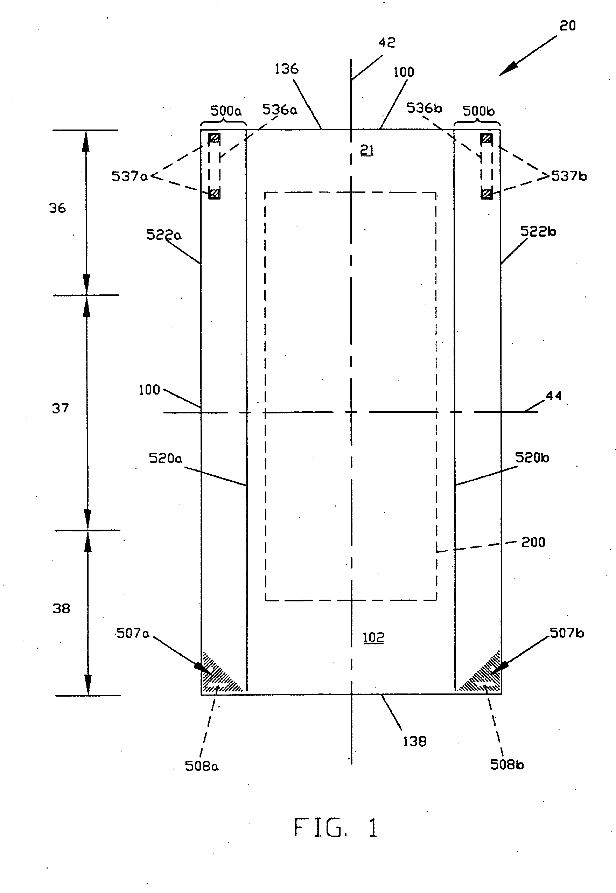

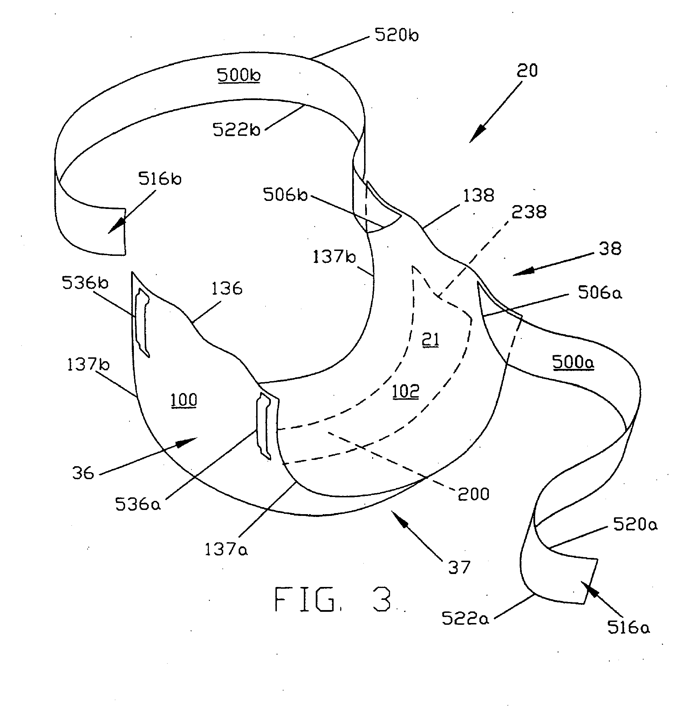

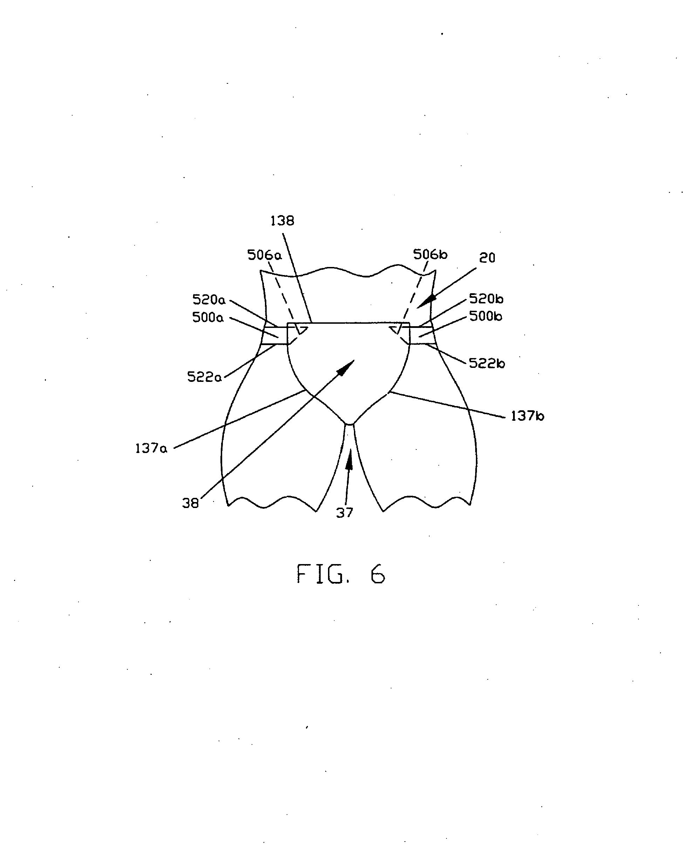

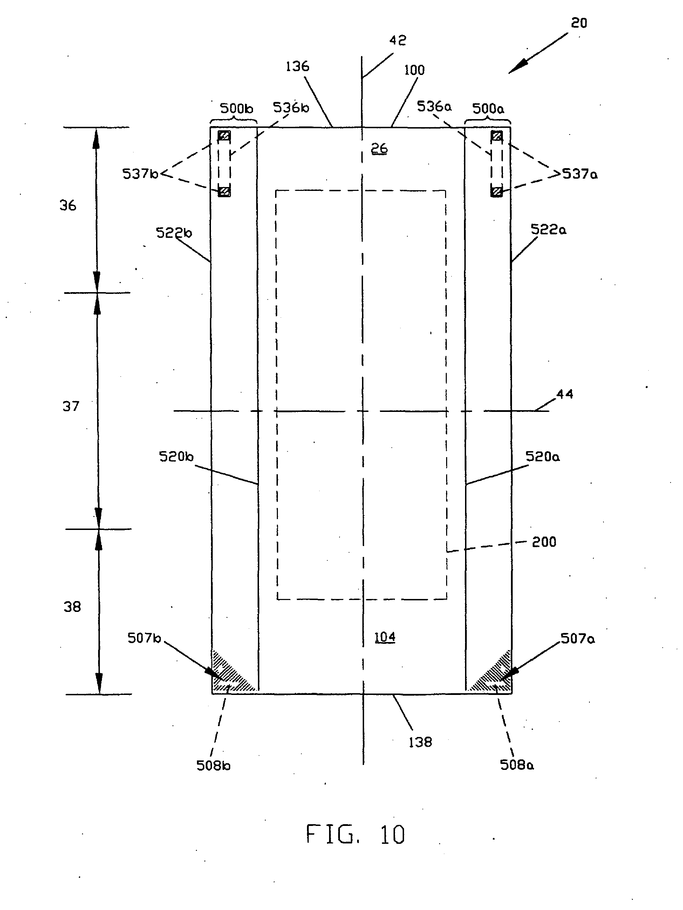

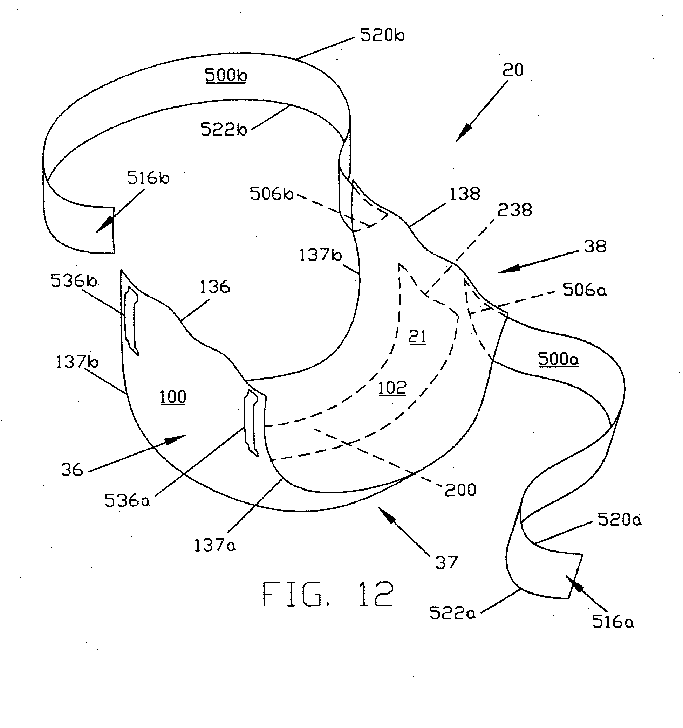

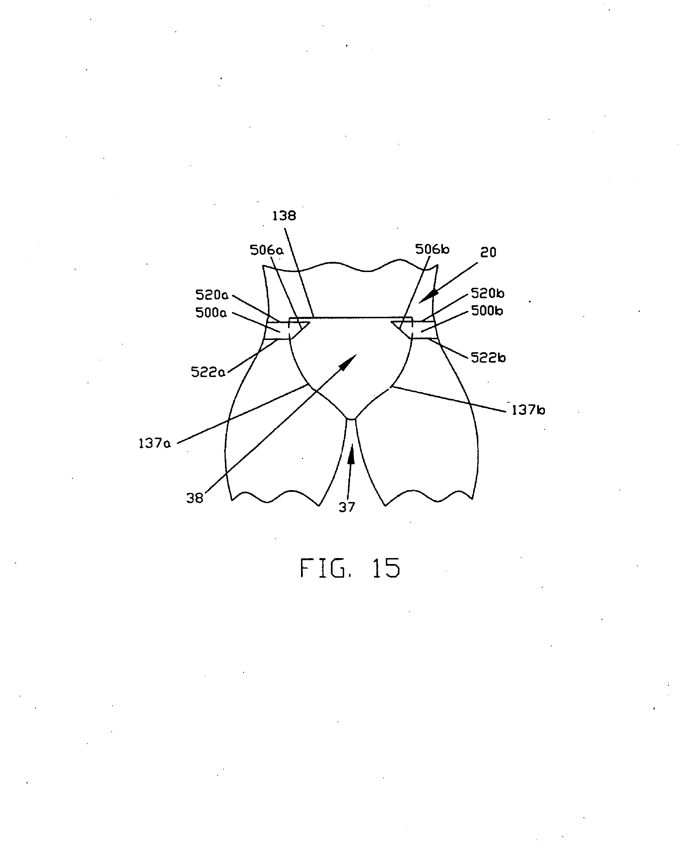

In **Figure 1** through **Figure 9****,** the belt strips **500** are shown disposed interiorly. Alternatively the belt strips **500** may be disposed exteriorly, as shown in **Figure 10** through **Figure 35****.**

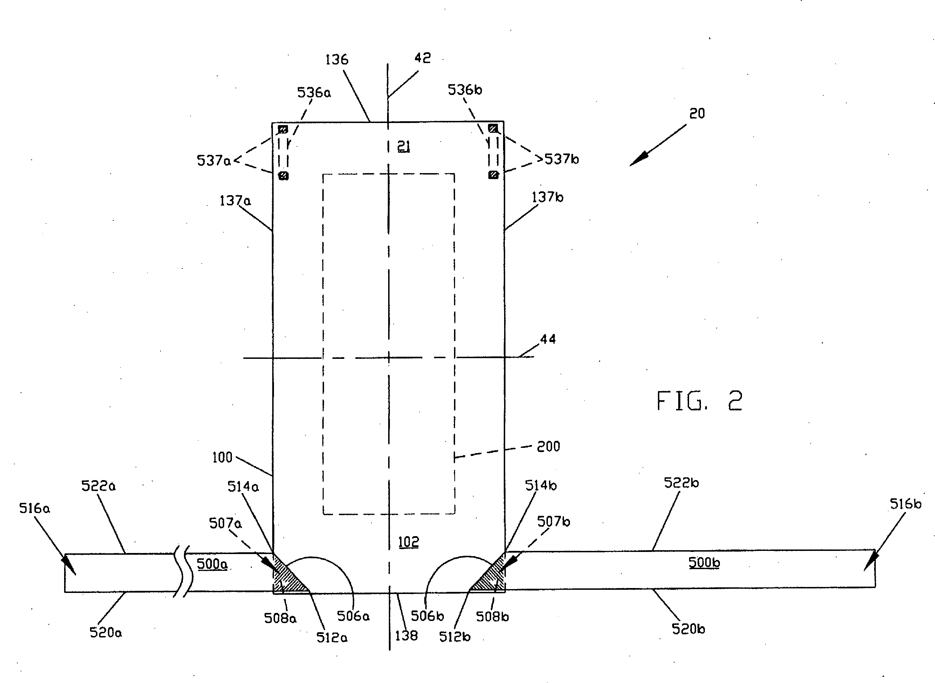

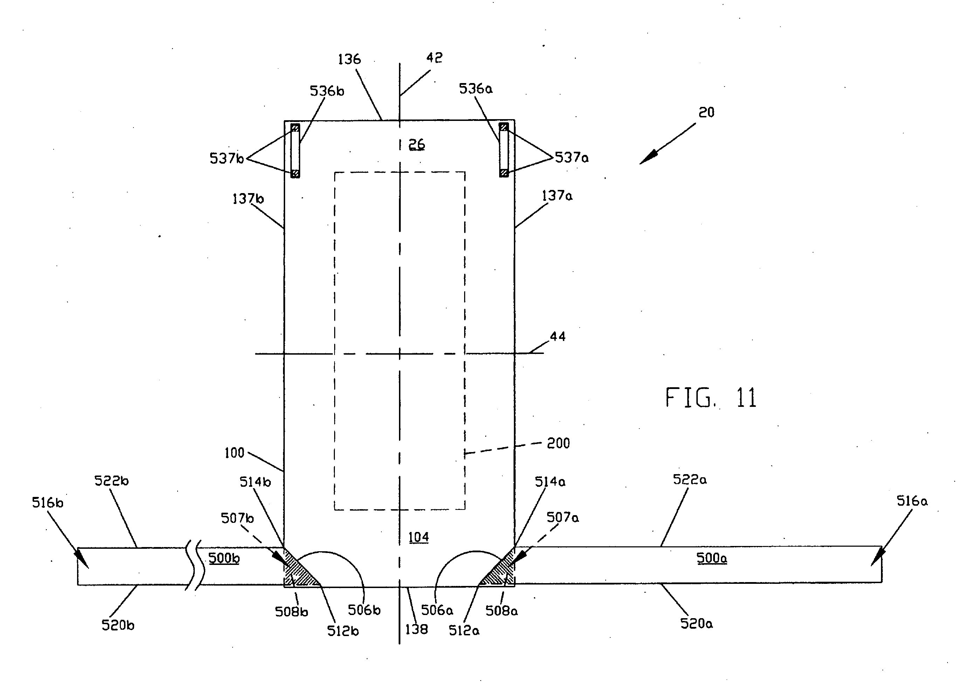

Each belt strip **500** is formed in an attached configuration as shown, for example, in **Figure 1** and in **Figure 10****.** The belt strip **500** is deployed for use by detaching the belt strip **500** except at its fixed end portion **507** and folding the belt strip **500** laterally outward at a diagonal fold line **506** as shown, for example, in **Figure 2** and in **Figure 11****.** Once deployed, each belt strip **500** is tied to another belt strip, fastened to a waist region of the diaper, and/or fastened to another belt strip in order to thereby partially or wholly encircle the waist of the wearer of the diaper **20.**

In the present figures, the diagonal fold lines **506** are located adjacent to the back waist edge **138** of the diaper **20** and the belt strips **500** extend from there toward the front waist edge **136.** Alternatively, the diagonal fold lines **506** may be located adjacent to the front waist edge **136** of the diaper **20,** in which configuration the belt strips **500** extend toward the back waist edge **138.** In general, other structural elements that are described in relation to the belt strips and whose disposition is dependent on the disposition of the belt strips may likewise be located oppositely in combination with oppositely disposed belt strips.

The belt strip **500** has a longitudinally extending first edge **520** and a laterally opposing longitudinally extending second edge **522.** Each of the first and second edges is formed by either an edge of a sheet of material, a fold in a sheet of material, or a frangible separation line. The first edge **520** is located laterally proximally relative to the second edge **522** prior to the deployment of the belt strip **500** for use. When the belt strip **500** is deployed for use, the first edge **520** is positioned as the upper edge and the second edge **522** is positioned as the lower edge of the belt strip **500,** *i.e.,* the first edge **520** is disposed farther from the lateral axis **44** than the second edge **522** is disposed.

The diagonal fold line **506** has a laterally proximal end point **512** and an opposing laterally distal end point **514** located longitudinally proximally relative to the laterally proximal end point **512.** In other words, the laterally distal end point **514** is located relatively closer to the lateral axis **44** of the diaper **20** than the laterally proximal end **point 512** is located.

When deployed for use, the upper edge **520** of the belt strip **500** extends laterally outward from the laterally proximal end point **512** and the lower edge **522** extends laterally outward from the laterally distal end point **514.** The laterally proximal end point **512** of the diagonal fold line **506** may be located at the respective waist edge or may be located below the waist edge, *i.e*., between the waist edge and the lateral axis **44.** Thus, when the laterally proximal end point **512** is located at the waist edge, the upper edge **520** of the belt strip **500** meets the waist edge. Similarly, when the laterally proximal end point **512** is located below the waist edge, the upper edge **520** of the belt strip **500** is likewise below the waist edge.

Any portion of the chassis **100** protruding longitudinally beyond the upper edge **520** of the deployed belt strip **500** is free to fold over, either interiorly or exteriorly. Such folding over may degrade the appearance of the diaper **20** on the wearer. In addition, this folding over may negatively affect the performance of the diaper. For example, folding over into the interior may undesirably expose an exterior layer of the diaper **20,** such as a plastic film, to the skin of the wearer. Conversely, folding over to the exterior may expose a wet interior layer of the diaper **20** to clothing or bedding. Therefore, it may be desirable to locate the laterally proximal end point **512** at or closely adjacent to the waist edge in order to minimize the size of any such portion of the chassis **100** protruding longitudinally beyond the upper edge **520** of the deployed belt strip **500** and thereby prevent, or at least minimize, the magnitude of any degradation in appearance and/or performance.

For the purpose of clarity in the present drawing figures, the laterally proximal end point **512** of each deployed belt strip **500** and the upper edge **520** of that deployed belt strip **500** are shown displaced slightly from the back waist edge **138** of the diaper **20,** rather than being shown exactly coincident with that waist edge. This depiction is intended to represent the preference that the upper edge **520** of the deployed belt strip **500** be located either at or closely adjacent to the waist edge in order to minimize the protrusion of the chassis **100** beyond the upper edge **520,** for the reason explained above. In order to locate the upper edge **520** as preferred, the distance between the laterally proximal end point **512** and the closest waist edge is less than the longitudinal distance between the laterally proximal end point **512** and the laterally distal end point **514.** For example, the laterally proximal end point **512** may be located within approximately 6 mm of the closest waist edge of the diaper **20.**

The diagonal fold line **506** may be oriented such that a deployed belt strip **500** extends parallel to the lateral axis **44** or at an angle with respect to the lateral axis **44.** For example, a belt strip **500** formed parallel to the longitudinal axis **42** and deployed by being folded laterally outward at a fold line **506** oriented at 45 degrees to both the longitudinal axis **42** and the lateral axis **44** of the diaper **20** extends parallel to the lateral axis **44** when deployed. However, when such a longitudinally parallel formed belt strip **500** is folded at a fold line **506** oriented at an angle other than 45 degrees, the belt strip **500** extends at an angle with respect to the lateral axis **44.** Similarly, a belt strip **500** formed at an angle relative to the longitudinal axis **42** and deployed by being folded laterally outward at a 45 degree diagonal fold line **506** extends at an angle with respect to the lateral axis **44.** For example, in some embodiments, it may be desirable to fit the deployed belt strip **500** on the torso of a wearer along a path running from the small of the back to below the navel.

In its fixed end portion **507,** the belt strip **500** is attached to another layer of the diaper **20** at both the laterally proximal end point **512** and the laterally distal end point **514** of the diagonal fold line **506** in an attachment zone **508.** The attachment zone **508** may have a continuous or intermittent form, for example two points, a pattern of more than two points, a continuous area, or a pattern of discontinuous areas. Thus, the belt strip **500** may be attached either continuously or intermittently along the diagonal fold line **506** between the laterally proximal end point **512** and the laterally distal end point **514.** The attachment zone **508** may be formed by any means suitable for the materials involved, including stitching, adhesive bonding, thermal bonding, stapling, and riveting, for example.

For example, as shown in **Figure 1** and in **Figure 10****,** the attachment zone **508** may extend longitudinally and laterally outward from the diagonal fold line **506** in directions away from both the longitudinal axis **42** and the lateral axis **44.** Such a triangular attachment zone **508** may be desirable in order to strengthen and/or stabilize this area where any force exerted by a deployed belt strip **500** is transmitted to the remainder of the structure of the diaper **20.**

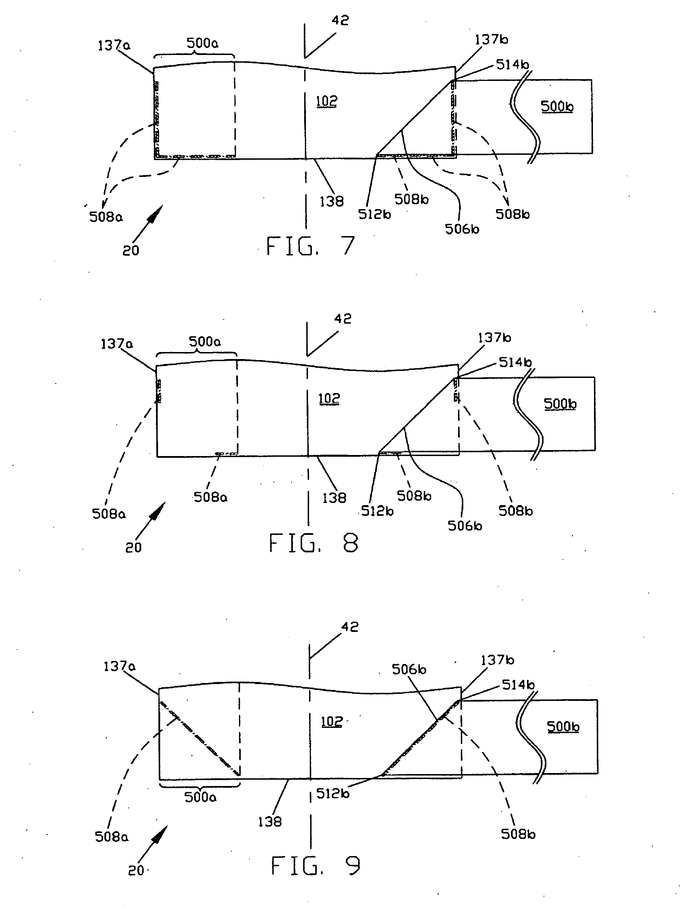

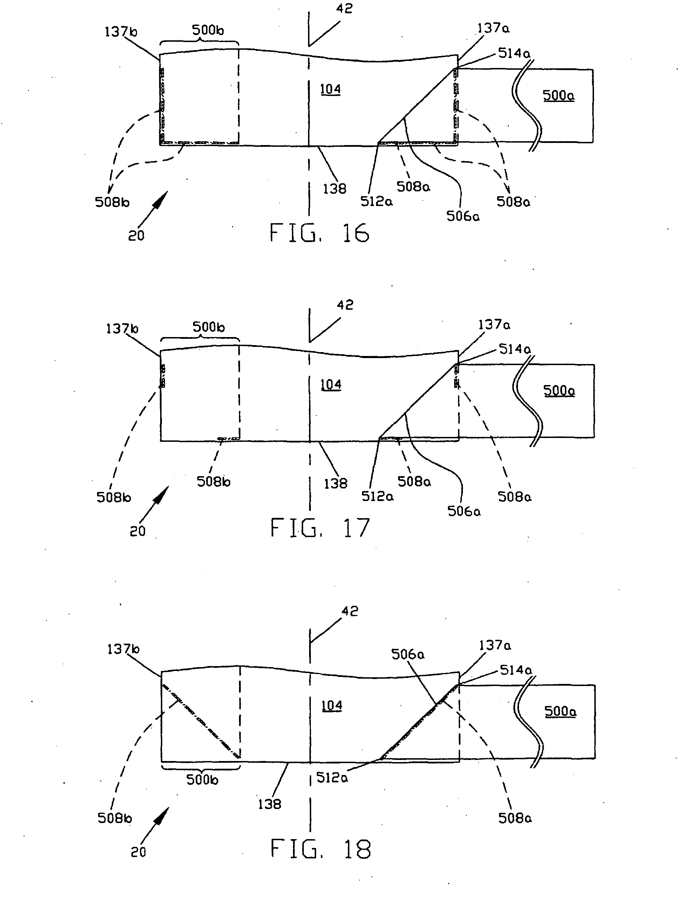

As other examples, as shown in **Figure 7** and **Figure 8** and in **Figure 16** and **Figure 17****,** the attachment zone **508** may extend longitudinally from the laterally distal end point **514** of the diagonal fold line **506** in a direction away from the lateral axis **44** toward or to the adjacent waist edge of the diaper **20** and laterally from the laterally proximal end point **512** of the diagonal fold line **506** in a direction in a direction away from the longitudinal axis **42** toward or to the adjacent side edge of the diaper **20,** without forming a triangle. As yet another example, as shown in **Figure 9** and in **Figure 18****,** the attachment zone **508** may extend from the laterally proximal end point **512** toward or to the laterally distal end point **514** along the diagonal fold line **506** itself.

Because the belt strip **500** is attached at least at both ends of the diagonal fold line **506,** any tension in the belt strip **500** is transmitted to the remainder of the structure of the diaper **20** over the width of the belt strip **500,** rather than being concentrated at a single point. Such a distributed transmission of force may be desirable in order to minimize the possibility of marking the skin of the wearer and/or to minimize the possibility of overstressing the structure. In particular, when the belt strip **500** is attached along the entire diagonal fold line **506** or in a triangular attachment zone **508** as described above, the tensile force may be uniformly distributed across the width of the belt strip **500.**

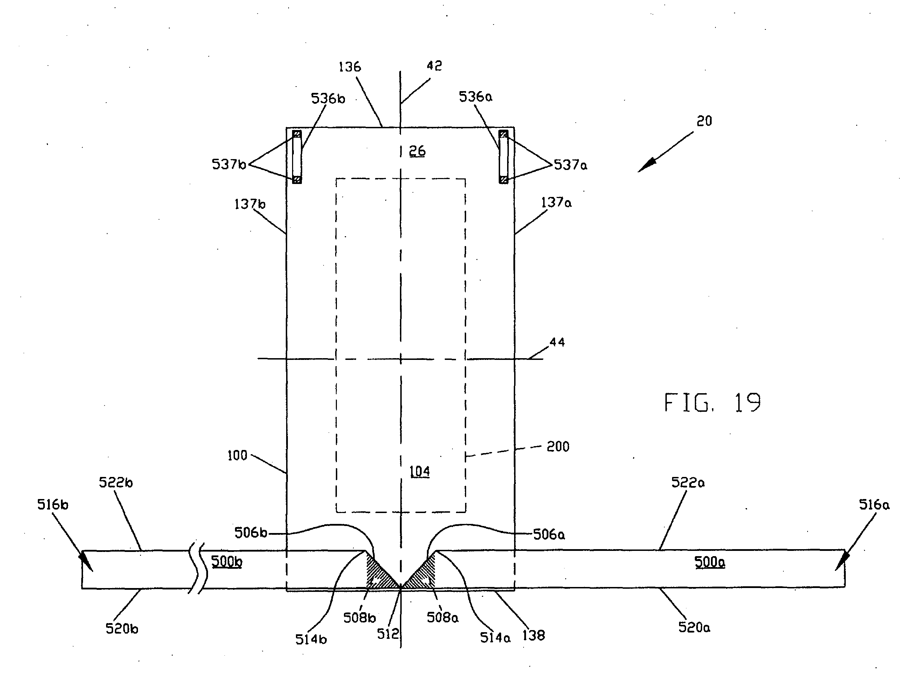

In **Figure 1** through **Figure 18****,** the diaper **20** has two belt strips **500** that are laterally spaced apart. Alternatively, two belt strips **500** may be laterally abutted, rather than being spaced apart. For example, in **Figure 19****,** the two belt strips **500** are disposed such that their respective first edges **520** extend from a common laterally proximal end point **512** of both of their diagonal fold lines **506.** Thus, prior to deployment, these two belt strips **500** had a common first edge **520** extending from the common laterally proximal end point **512.** In **Figure 19****,** the two belt strips **500** are disposed symmetrically with respect to the longitudinal axis **42** of the diaper **20.** Alternatively, two laterally abutted belt strips **500** may be disposed asymmetrically with respect to the longitudinal axis **42** of the diaper **20.**

Prior to deployment for use, each belt strip **500** may extend from the laterally proximal end point **512** of the diagonal fold line **506** to the opposing waist edge. For example, in **Figure 1** and in **Figure 10****,** each belt strip **500** extends from its laterally proximal end point **512** located adjacent to the back waist edge **138** to the opposing front waist edge **136.** When such a "full length" belt strip **500** is deployed for use, a portion of the opposing waist edge defines a free end portion **516** of the belt strip **500,** as shown in **Figure 2****,** **Figure 3****,** **Figure 11****,** and **Figure 12****.**

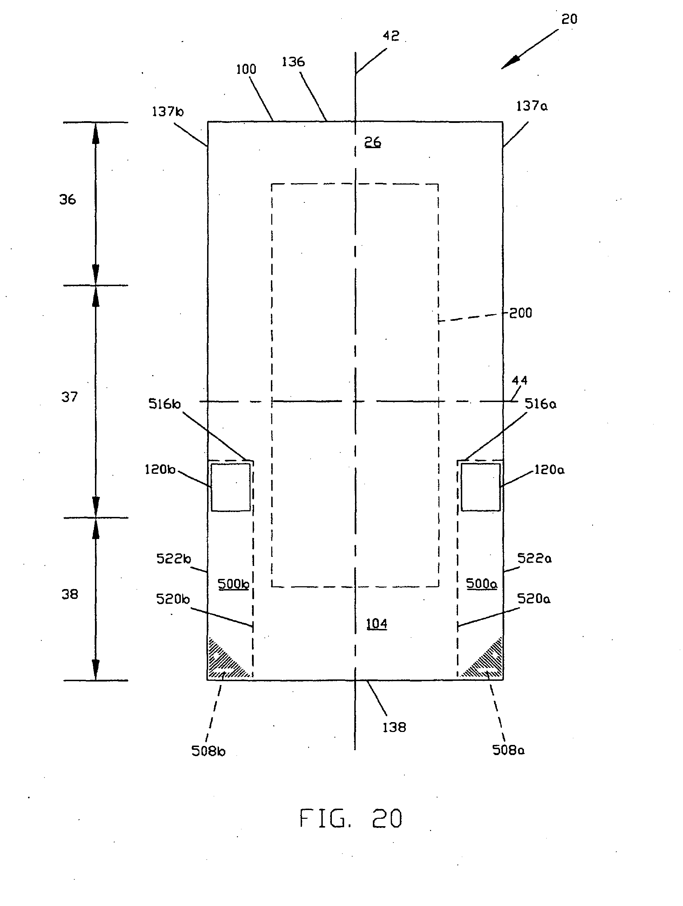

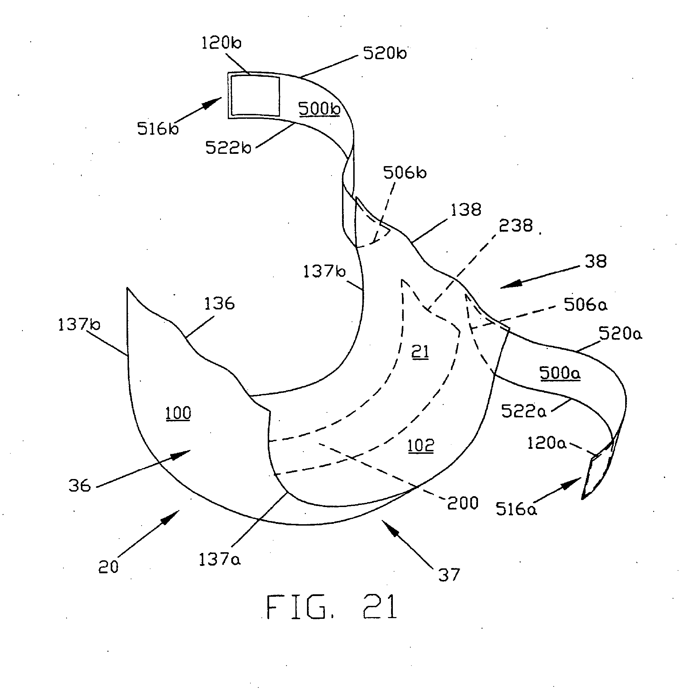

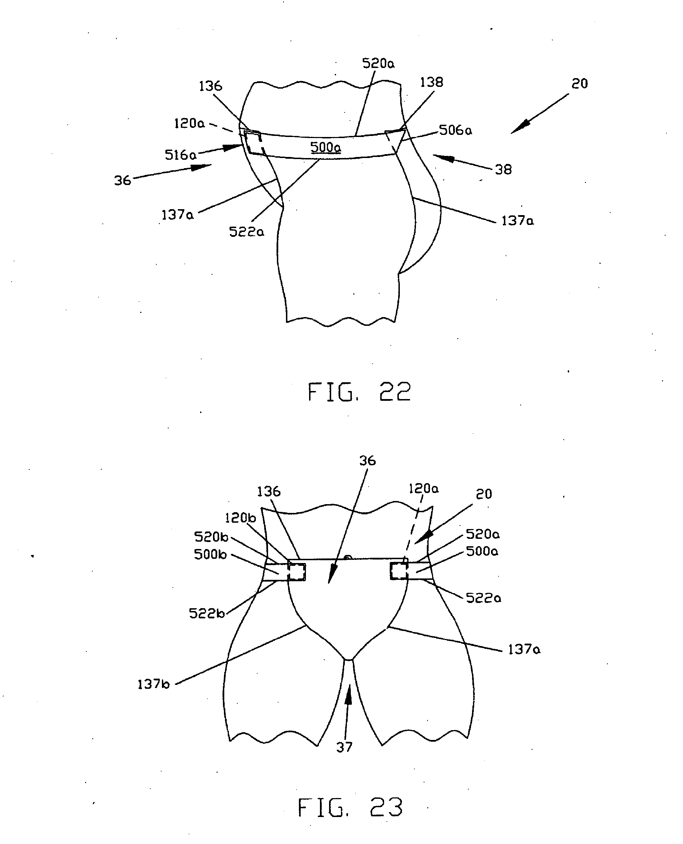

Alternatively, the belt strip **500** may extend only a part of the way between the laterally proximal end point **512** and the opposing waist edge. For example, in **Figure 20** and in **Figure 21****,** each belt strip **500** extends from its laterally proximal end point **512** located adjacent to the back waist edge **138** to a free end portion **516** located between the laterally proximal end point **512** and the opposing front waist edge **136.** This free end **516** may be defined by a laterally extending frangible separation line. As shown in **Figure 22** and **Figure 23****,** when the diaper **20** is applied onto the wearer, each such partial length belt strip **500** may be used to connect the waist regions at and/or adjacent to a respective side edge of the diaper **20.** Such laterally opposing partial length belt strips **500** may overlap or may end short of overlapping.

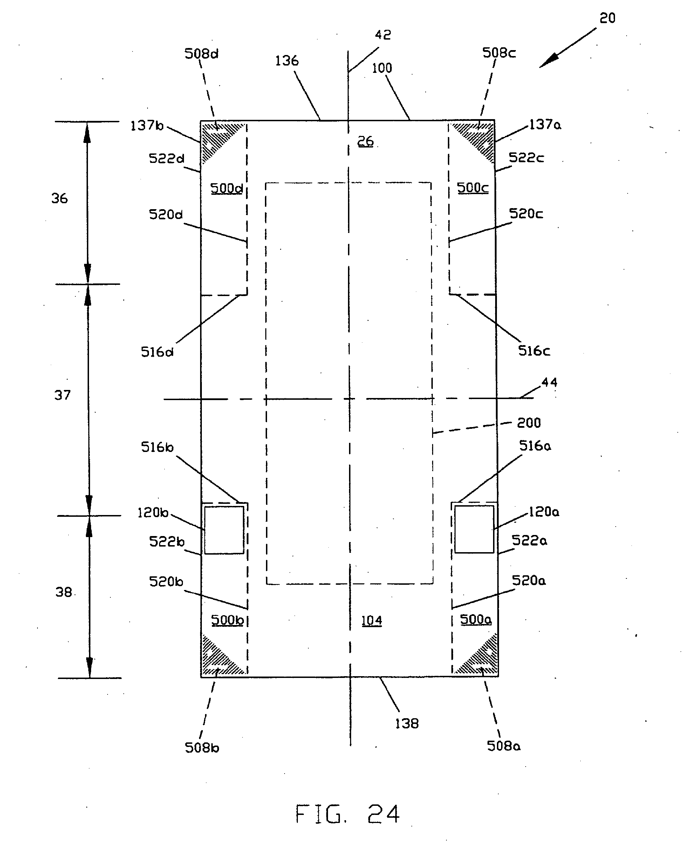

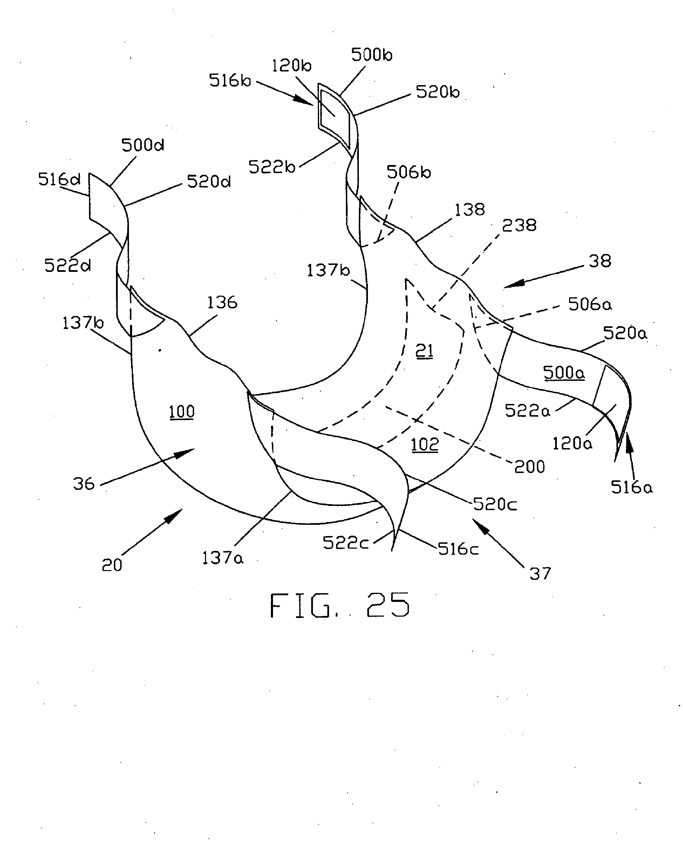

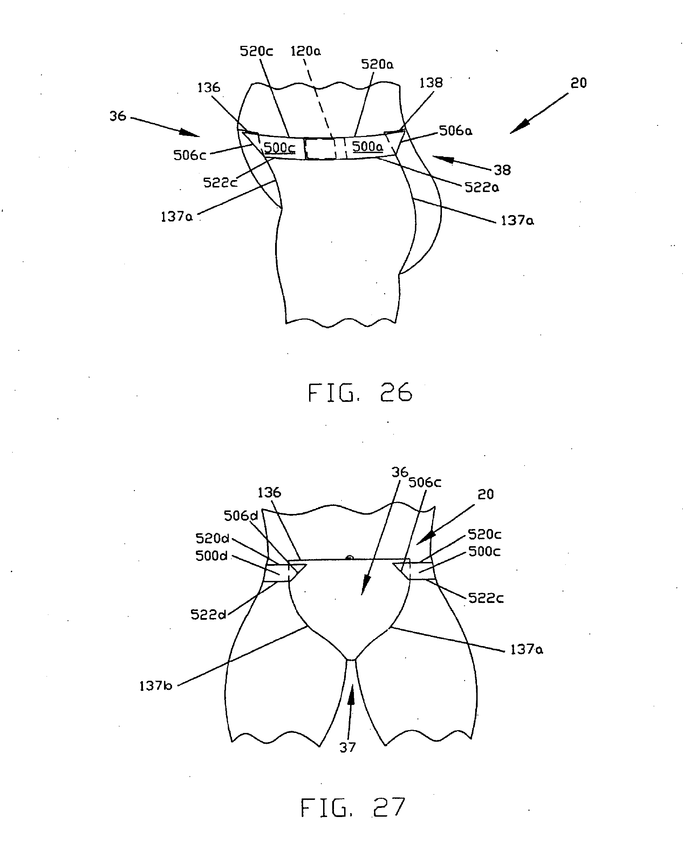

As another alternative, as shown in **Figure 24** and in **Figure 25****,** two longitudinally opposing partial length belt strips **500** may be formed adjacent to each side edge of the diaper **20,** for a total of four belt strips **500.** When the diaper **20** is applied onto the wearer, the two laterally opposing partial length belt strips **500c** and **500d** in the front waist region **36** and the respective laterally opposing partial length belt strips **500a** and **500b** in the back waist region **38** are used to connect the waist regions at and/or adjacent to the respective side edges of the diaper **20** as shown in **Figure 26** and in **Figure 27****.** In particular, the partial length belt strips **500a** and **500c** adjacent to the left side edge **137a** are attached together and the partial length belt strips **500b** and **500d** adjacent to the right side edge **137b** are attached together.

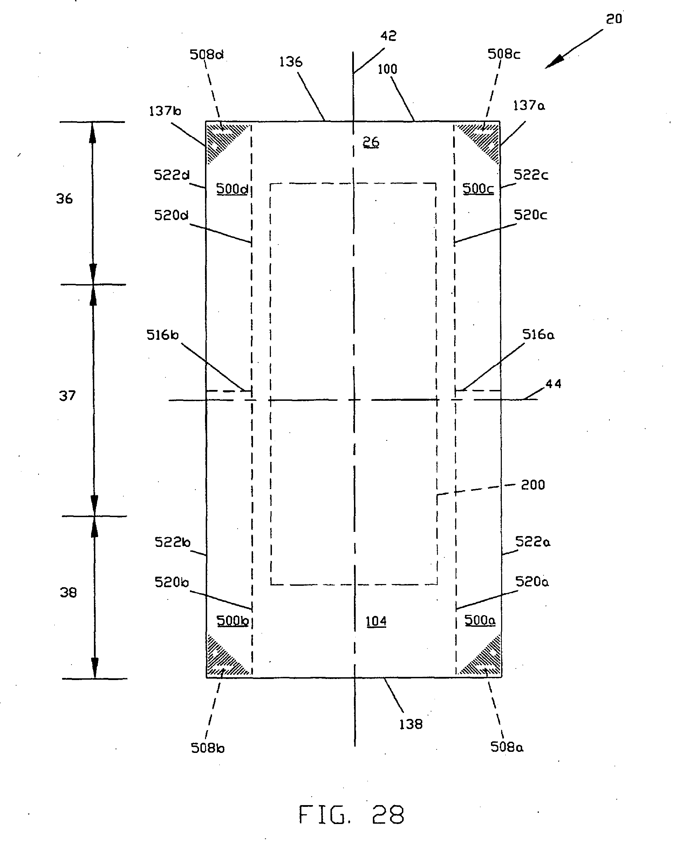

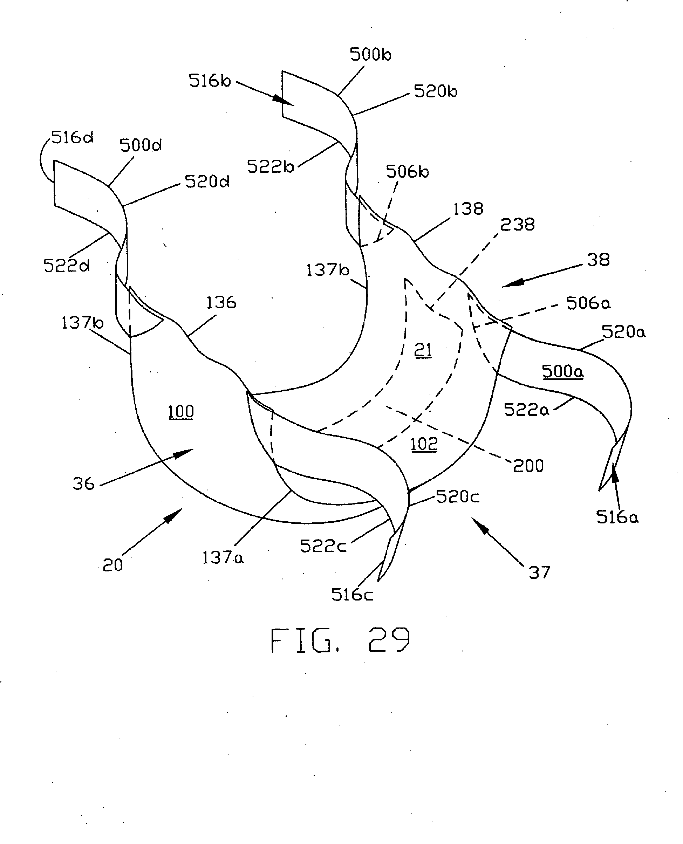

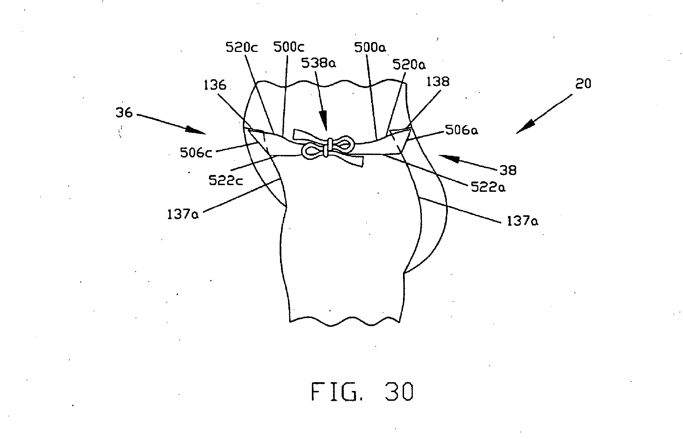

As shown in **Figure 24****,** the two longitudinally opposing partial-length belt strips **500** on each side may not meet. As an alternative, as shown in **Figure 28** and in **Figure 29****,** the two longitudinally opposing partial length belt strips **500** on each side may meet at their free end portions **516,** thereby being relatively longer than in a configuration in which they do not meet, and may be long enough to be tied together, as shown in **Figure 30****.**

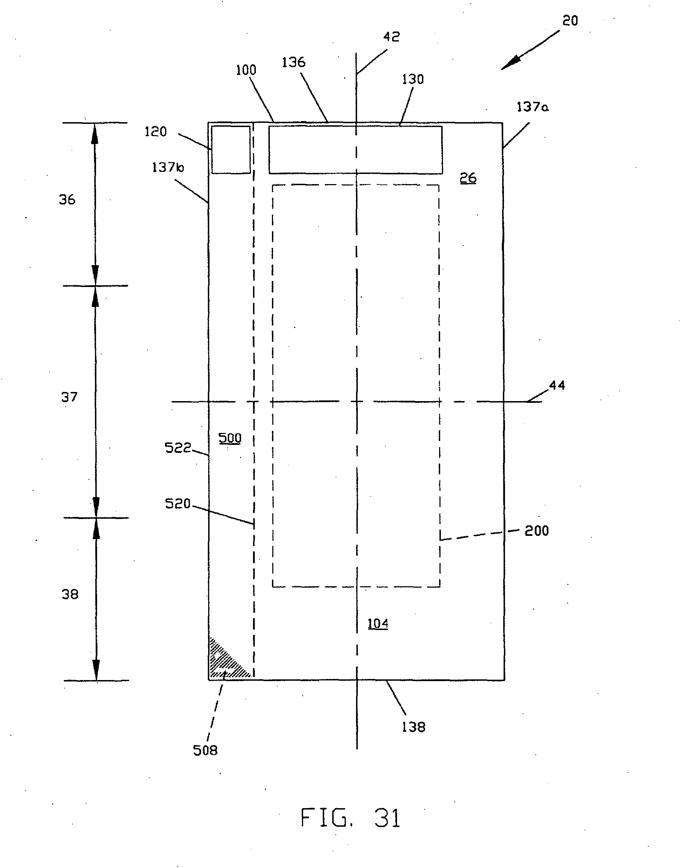

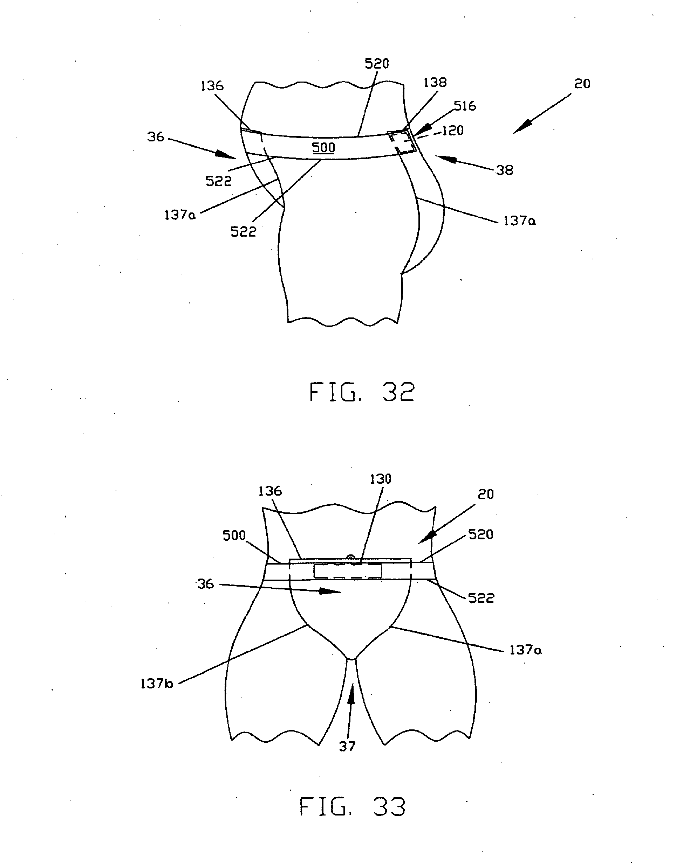

As another alternative, as shown in **Figure 31** through **Figure 34****,** the diaper **20** may have only a single deployable belt strip **500.** When the diaper **20** is applied onto the wearer, such a "full length" belt strip **500** may be long enough to extend across the entirety of the opposing waist region and back to the starting waist region. In other words, a single full length belt strip **500** may be used to connect the waist regions at and/or adjacent to both of the side edges of the diaper **20.** For example, as shown in **Figure 32, Figure 33****,** and **Figure 34****,** the single belt strip **500** in **Figure 31** extends from the diagonal fold line **506** adjacent to the right side edge **137b** in the back waist region **38** across the front waist region **36** and to the back waist region **38** such that its free end portion **516** lies adjacent to the left side edge **137a** in the back waist region **38.**

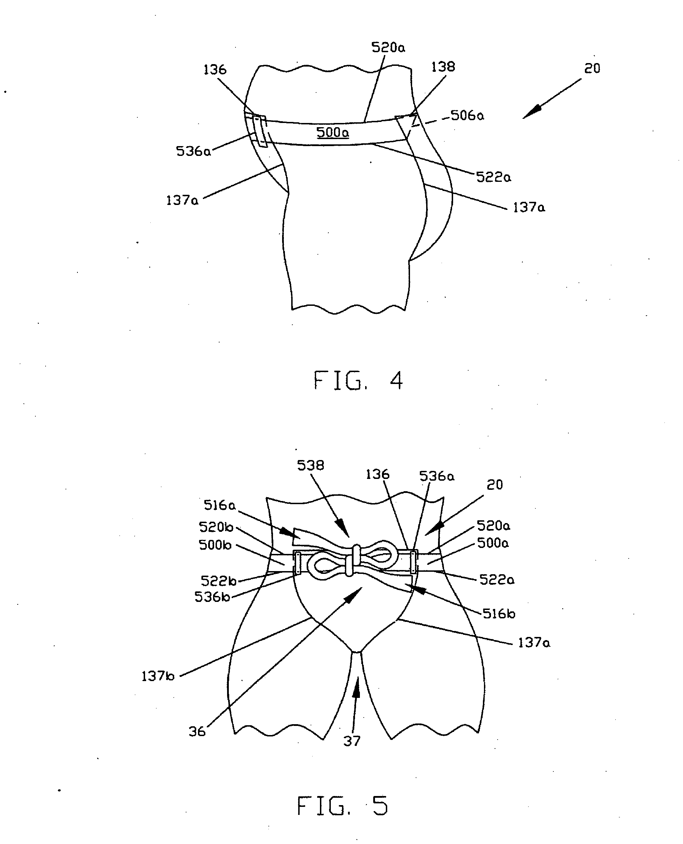

As shown in **Figure 5****,** **Figure 6****,** **Figure 14****,** and **Figure 30****,** two deployed belt strips **500** may be tied together in a knot **538** when they are long enough to make this practical. Alternatively, a fastener may be used to attach two deployed belt strips **500** together. Prior to fastening, the fastener may be disposed on either of the two belt strips **500.** For example, in **Figure 26****,** the fastener **120a** is used to attach the back left belt strip **500a** to the front left belt strip **500c** and the fastener **120b** is similarly used to attach the back right belt strip **500b** to the front right belt strip **500d.** Alternatively, complementary fasteners may be disposed on matching belt strips **500,** *e.g*., a hook patch may be disposed on one belt strip and a complementary loop patch may be disposed on another belt strip such that the two belt strips may be fastened together.

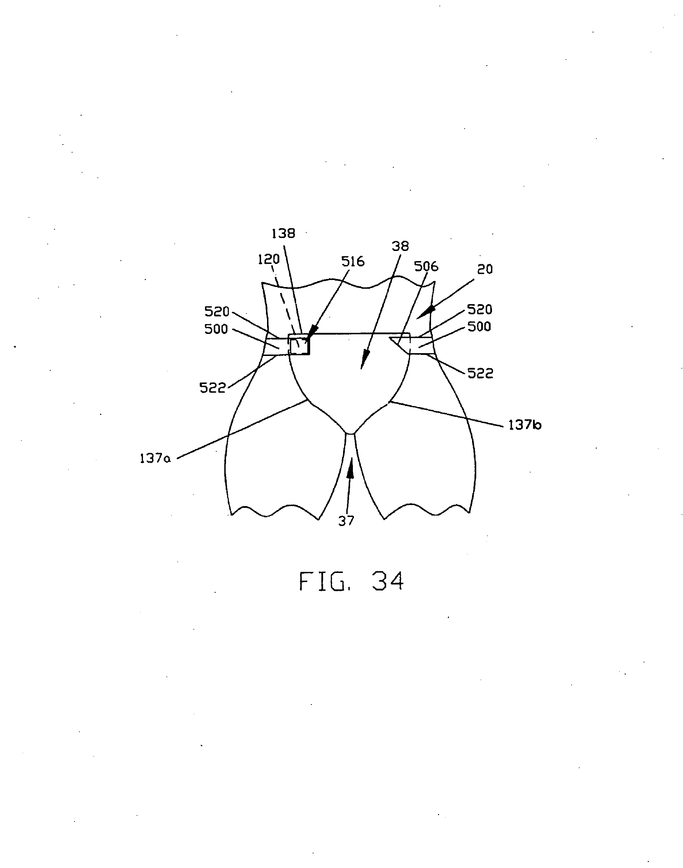

Alternatively, a fastener may be used to attach a deployed belt strip **500** to another portion of the diaper **20.** Prior to fastening, the fastener may be disposed on the belt strip **500** or may be disposed on the other portion of the diaper **20** to which the belt strip **500** is to be fastened. For example, in **Figure 22** and **Figure 23****,** each fastener **120** is used to attach the respective belt strip **500** to the front waist region **36** of the diaper **20.** As another example, in **Figure 32** and **Figure 34****,** the single fastener **120** is used to attach the single belt strip **500** to the back waist region **38.** Alternatively, complementary fasteners may be used, *e.g*., a hook patch may be disposed on a belt strip and a complementary loop patch may be disposed on the other portion of the diaper **20** to which the belt strip is to be fastened.

The fastener **120** may be any type of fastening device suitable for the materials involved, for example an adhesive fastener, a cohesive fastener, a hook, a loop, a button, a patch of hooks, a patch of loops, *etc.* A fastener in the form of a patch of hooks that engage a nonwoven material may be suitable in some embodiments. The fastening of the belt strip **500** may become permanent once it is made, such that it cannot be undone without damage to the structural elements involved. Alternatively, the fastening of the belt strip **500** can be releasable and refastenable, such that it can be released for adjustment or for inspection of the interior of the diaper **20** and then refastened as before. The belt strip **500** may be fastened and/or tied at and/or adjacent to its free end portion **516.** Alternatively or in addition, the belt strip **500** may be fastened and/or tied at one or more intermediate points between the diagonal fold line **506** and the free end portion **516.**

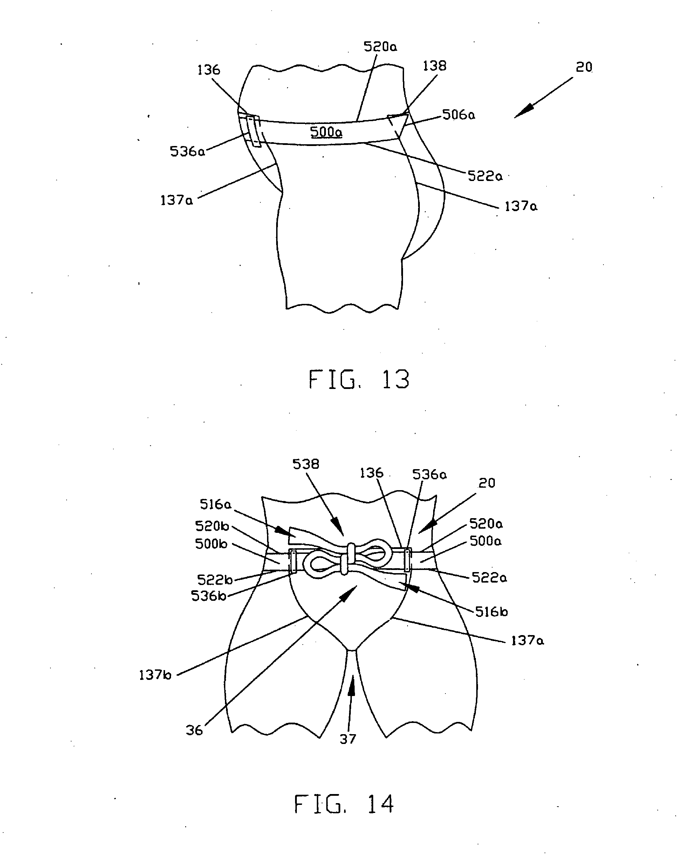

When a deployed belt strip **500** is attached to a waist region by a fastener **120** or when two deployed belt strips **500** are tied or fastened together at a side of the body as in **Figure 30****,** both waist regions of the diaper **20** will be supported by the belt strips **500** that are attached to them. However, when two deployed belt strips **500** are tied together over a waist region to which they are not attached, as in **Figure 5** and in **Figure 14****,** or when a deployed belt strip **500** passes completely across a waist region to which it is not attached, as in **Figure 33****,** that waist region may tend to slide downward, *i.e*., toward the crotch region **37,** relative to the belt strip **500,** depending on the coefficients of static and dynamic friction between the waist region and the belt strip **500.** In some embodiments, this inherent friction may be sufficient to prevent relative movement. Alternatively, it may be necessary and/or desirable to supplement such inherent friction in order to ensure that the waist region will not slide downward.

For example, in **Figure 1** through **Figure 5****,** in **Figure 10** through **Figure 14****,** and in **Figure 19****,** the belt strips **500** pass through laterally spaced belt loops **536,** each of which is attached to the front waist region **36.** Each belt loop **536** transfers force from the waist region to the belt strip **500** and thereby supports the front waist region **36** from the belt strip **500.** The belt loops **536** may be attached to the waist region by any means suitable for the materials involved, including stitching, adhesive bonding, thermal bonding, stapling, and riveting, for example. For example, the belt loops **536** in the present figures are shown attached in attachment zones **537.**

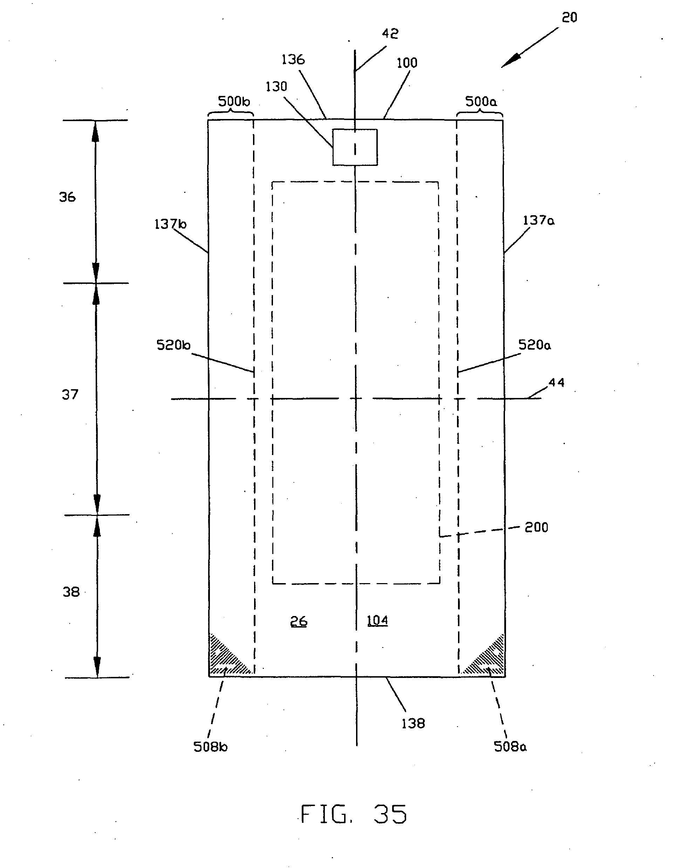

Optionally, as shown in **Figure 31** **Figure 33****,** and **Figure 35****,** an additional fastener **130** similar to any of the fasteners **120** described above may be disposed on the front waist region **36,** where it will be overlapped by a belt strip **500,** in order to transfer force from the waist region to the belt strip **500** and thereby support the waist region from the belt strip **500.** Such a fastener may be disposed on the belt strip **500,** instead of on the chassis **100** as shown. A suitable fastener may be relatively wide as shown in **Figure 31** and **Figure 33** or relatively narrow as shown in **Figure 35** and may have any shape, such as the rectangular shape shown in these figures. As an alternative to a fastener, a friction patch having a relatively high coefficient of static friction may be used. The fastener or friction patch may be disposed such that the belt strip may be overlapped exteriorly of the waist region, as shown in the figures. Alternatively, the fastener or friction patch may be disposed such that the belt strip lies against the body of the wearer and the waist region is overlapped exteriorly of the belt strip.

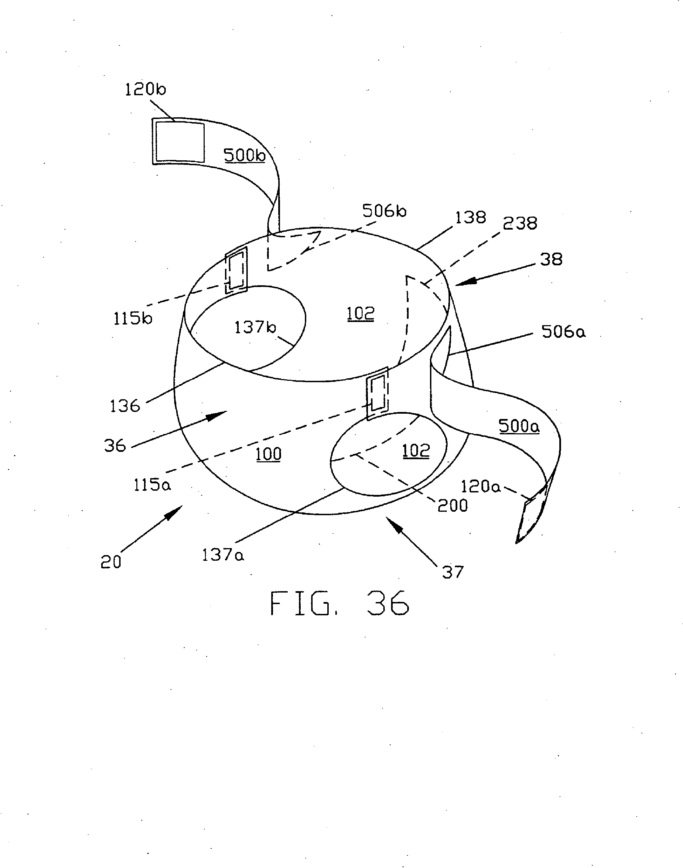

As shown in **Figure 36****,** an exemplary diaper **20** having exteriorly disposed belts strips **500** may have side seams **115** at which the front and back waist regions **36** and **38** are non-releasably attached together adjacent to the respective side edges **137** and thereby have the form of pants. In such an embodiment, the belt strips **500** can be used to set and/or adjust the tightness around the waist of the wearer.

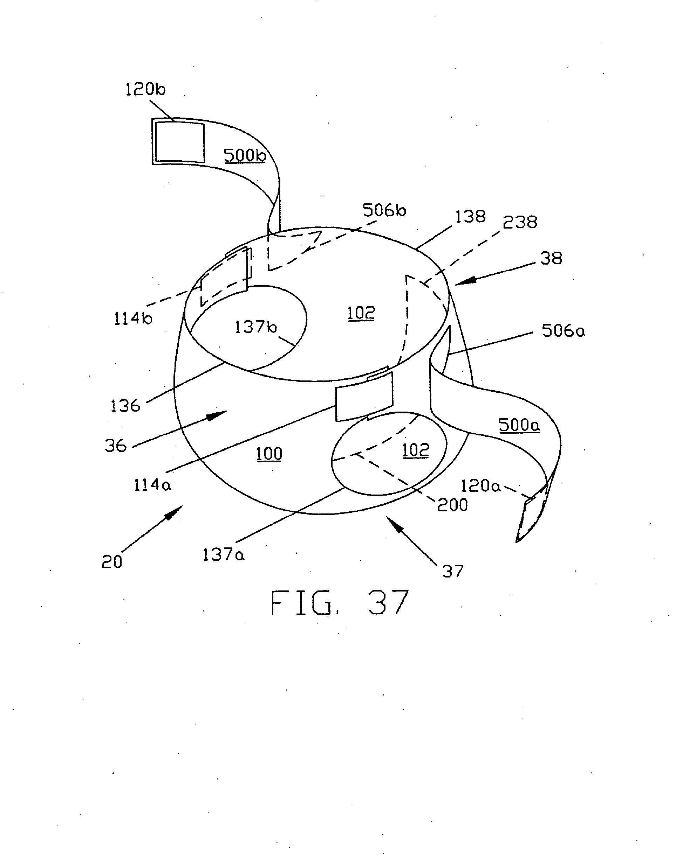

Similarly, as shown in **Figure 37****,** an exemplary diaper **20** having exteriorly disposed belts strips **500** may have side fasteners **114** by which the front and back waist regions **36** and **38** are attached together adjacent to the respective side edges **137.** The side fasteners **114** may be releasable and refastenable, thereby allowing for easy inspection of the interior of the diaper **20** while it is being worn and subsequent refastening when it is not necessary to change the diaper. The diaper **20** may be provided to the user with the side fasteners **114** already fastened or in an unfastened condition.

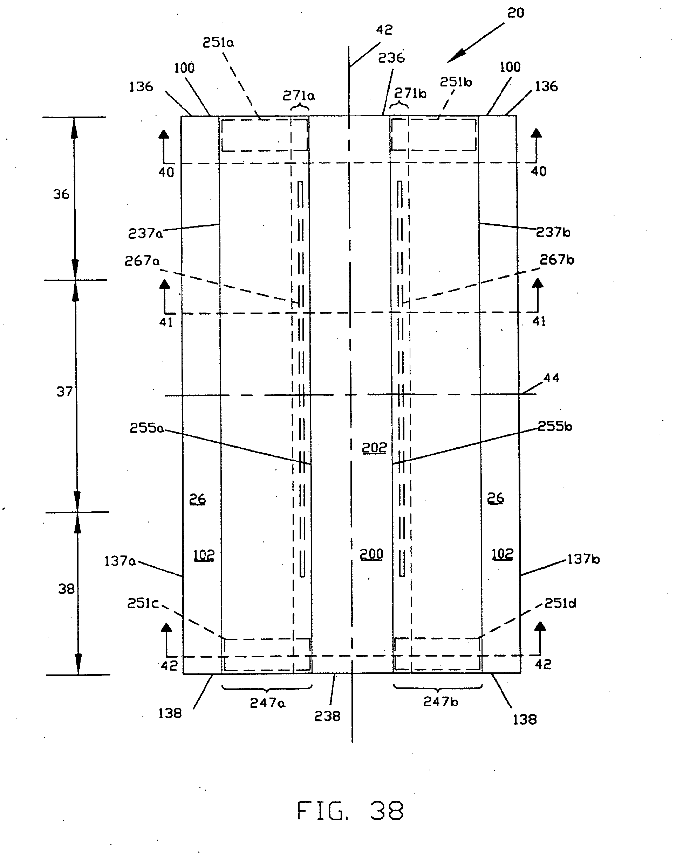

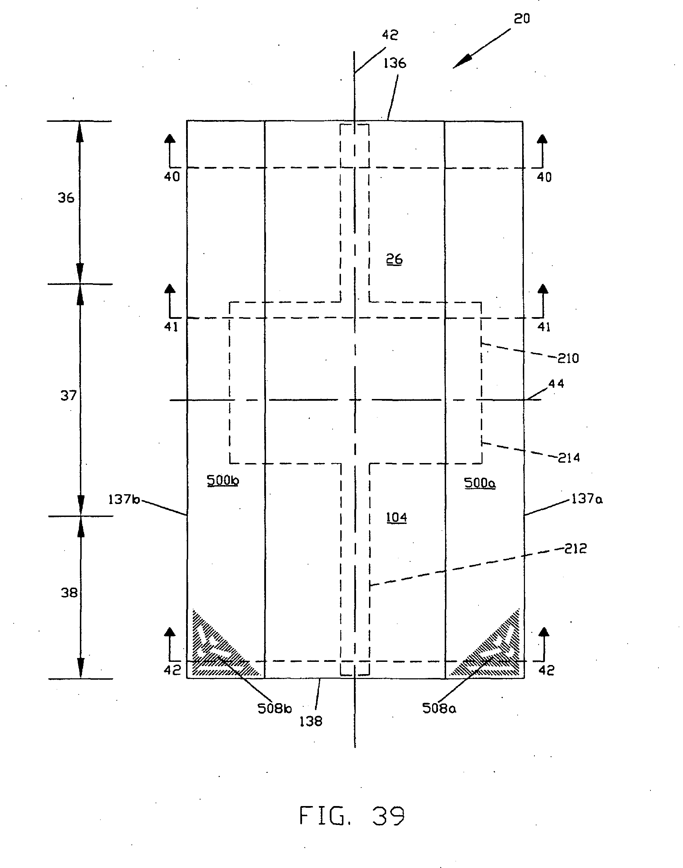

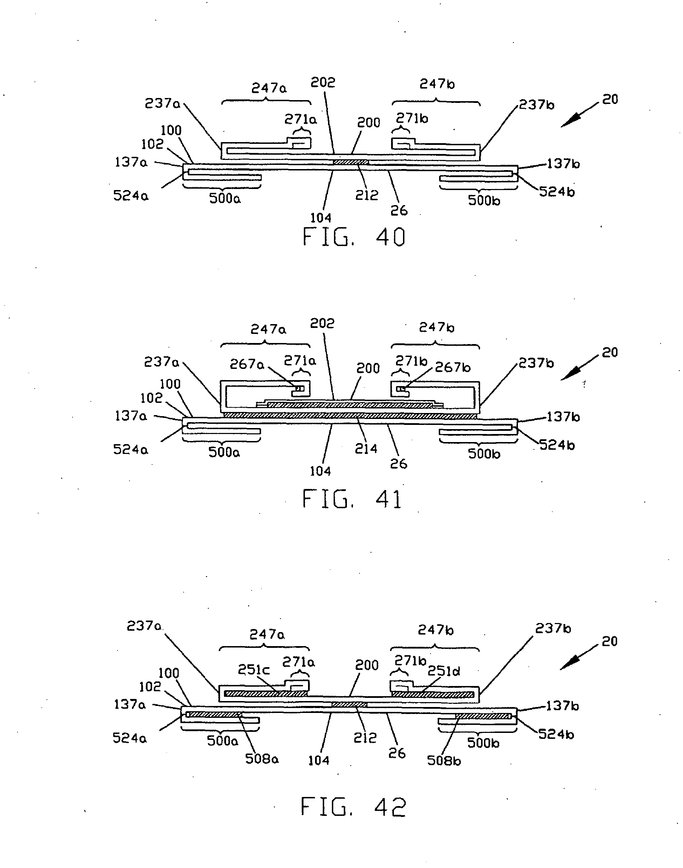

The exemplary diaper **20** in **Figure 38** through **Figure 42** has a structure in which an absorbent assembly **200** is attached to an interior surface **102** of a chassis **100.** The chassis **100** includes a water-impermeable backsheet **26.** The backsheet **26** forms an exterior surface that is intended to be placed toward any clothing that is worn over the diaper **20.** Many suitable materials for use as the backsheet **26** are well-known, including films of polyethylene and other polyolefins. Multi-layer backsheets, such as laminates of a film and a nonwoven, are also well-known and may be suitable for use as the backsheet **26.** Such a laminate backsheet may be oriented with the nonwoven disposed exteriorly to provide the feel and appearance of a more cloth-like outermost layer than would be provided by using the film as the outermost layer.

As shown in the figures, the absorbent assembly **200** has a laterally extending front edge **236** in the front waist region **36** and a longitudinally opposing and laterally extending back edge **238** in the back waist region **38.** The absorbent assembly **200** also has laterally opposing side edges **237** extending longitudinally between the front edge **236** and the back edge **238.** In addition, the exemplary absorbent assembly **200** has longitudinally extending and laterally opposing side flaps **247** that are disposed on the interior portion of the diaper **20** that faces inwardly toward the wearer and contacts the wearer. The side flaps **247** are formed by folding portions of the absorbent assembly **200** laterally inward, *i.e*., toward the longitudinal axis **42,** to form both the respective side flaps **247** and the side edges **237** of the absorbent assembly **200,** as shown in the figures

Each side flap **247** is attached to the interior surface **202** of the absorbent assembly **200** in attachment zone **251** adjacent to the front waist edge **236** and in a longitudinally opposing attachment zone **251** adjacent to the back waist edge **238.** Between the attachment zones, the proximal edge **255** of the side flap **247** remains free, *i.e.,* not attached to the interior surface **202** of the chassis **100** or to the absorbent assembly **200.** Also between the attachment zones, an elastic strand **267** is attached adjacent to the proximal edge **255** of each side flap **247.** Each elastic strand **267** is enclosed inside a hem **271** formed adjacent to the proximal edge **255** of each side flap **247.** When stretched, the elastic strand **267** allows the adjacent side flap edge to extend to the flat uncontracted length of the absorbent assembly **200.** When allowed to relax, the elastic strand **267** contracts to gather the portion of the adjacent side flap edge and thereby bend the diaper **20** into a "U" shape in which the interior of the "U" shape is formed by the portions of the diaper **20** that are intended to be placed toward the body of the wearer. This lifting of the proximal edges **255** when the diaper **20** is in the relaxed condition lifts the side flaps **247** into position to serve as side barriers adjacent to the side edges **237** of the absorbent assembly **200.**

The absorbent assembly **200** may be attached to the chassis **100** over any part or the whole of the area of the absorbent assembly **200.** Preferably, the absorbent assembly **200** is attached to the chassis **100** in a cruciform attachment pattern **210,** *i.e.,* in an attachment pattern that forms or is arranged in a cross or "+" shape. The portions of the chassis **100** that lie outside such a cruciform attachment pattern are not restrained by attachment to the absorbent assembly **200** and therefore remain extensible. In particular, a relatively narrow longitudinally extending portion **212** of a cruciform attachment pattern **210** leaves the majority of the width of the chassis **100** in the front waist region **36** and in the back waist region **38** freely extensible and thereby allows extension of the chassis **100** in the lateral direction in these regions. A relatively wide laterally extending portion **214** of a cruciform attachment pattern **210** prevents the portion of the chassis **100** in the crotch region **37** to which the absorbent assembly **200** is attached from shifting relative to the absorbent assembly **200** in that region.

Several suitable configurations of chassis and absorbent assemblies are described in more detail in U.S. Patent Application Publication No. 2005/0203475 of 15 September 2005 and in U.S. Patent Applications Nos. 10/880,135 filed on 29 June 2004 and 11/159,916 filed on 23 June 2005.

A belt strip **500** may be formed contiguously with another structural element of the diaper **20.** At least one edge of such a contiguous belt strip **500** is defined by a frangible separation line along which the belt strip **500** can be partially detached for use. Such a frangible separation line may be formed in a layer or a laminate of layers by perforation, by the formation of a brittle area or areas at which the material will preferentially fracture when stressed, by the formation of a weaker area or areas at which the material will preferentially tear when stressed, by the formation of a friable area or areas at which the material will preferentially crumble when stressed and/or bent, or by any other method of providing frangibility that is suitable for the materials involved.

For example, in the diaper **20** shown in **Figure 38** through **Figure 42****,** the backsheet **26** is folded laterally inward and each exteriorly disposed belt strip **500** is formed from the same layer as the backsheet **26** by a laterally outboard frangible separation line **524.** As can be readily understood by reference to the preceding description of various configurations of belt strips, in this example, each frangible separation line **524** corresponds to the second edge **522** of the respective belt strip **500** and each belt strip **500** can be deployed for use by being partially detached along its frangible separation line **524** and then folded outward along its diagonal fold line (not shown), which is defined during the deployment by its attachment zone **508.** In addition to being attached along the frangible separation line **524,** if desired for reasons related to handling, packaging, or appearance prior to deployment of the belt strips **500,** each belt strip **500** may be releasably attached to another layer with which it is in face-to-face contact. For example, each belt strip **500** may be releasably attached to the backsheet **26** adjacent to its first edge **520** and/or adjacent to the front edge **36** of the diaper **20.**

Alternatively, a belt strip **500** may be formed discretely rather than contiguously with another element of the diaper **20.** A configuration in which the belt strip is discretely formed may be chosen, for example, when it is desired to use a particular material for the belt strip that is different from either the side flap material or the backsheet material.

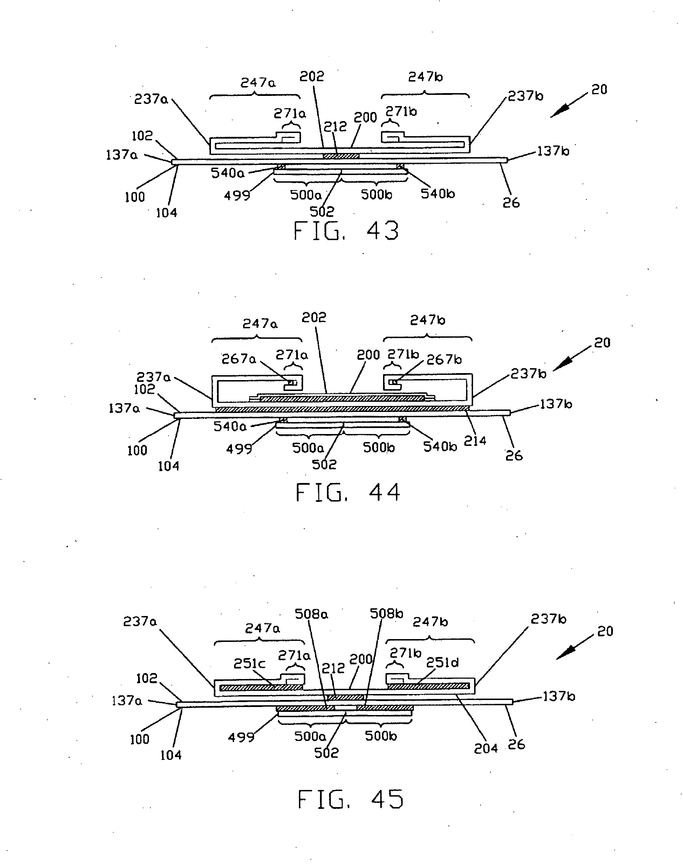

Such discretely formed belt strips may be disposed either interiorly or exteriorly. For example, a single discrete strip **499** is exteriorly attached to the backsheet **26** of the diaper **20** shown in **Figure 43** through **Figure 45** not showing embodiments of the present invention at longitudinally extending laterally spaced attachment zones **540**. A single common frangible separation line **502** located between the attachment zones **540** in this discrete strip **499** defines the two laterally abutted exteriorly disposed belt strips **500.** In this example, the belt strips **500** are releasably attached at the attachment zones **540** such that their deployment can be effected by detaching them there and at the frangible separation line **502**. Such a laterally abutted arrangement of the belt strips **500** may be desirable, for example, in order to concentrate the forces exerted by deployed belt strips on a relatively smaller area of the diaper **20** than is the case when the belt strips are laterally spaced apart. Also, in some embodiments, such laterally abutted exteriorly disposed belt strips may provide a relatively more finished appearance to the diaper **20** when it worn with the belt strips deployed around the waist of the wearer, due to the relatively greater extent of encirclement of the waist that is achievable with this configuration. Such a laterally abutted configuration of the belt strips may also be desirable in order to relatively simplify the process for manufacturing the diaper **20** by requiring only a single discrete strip **499** for the formation of two belt strips **500.**

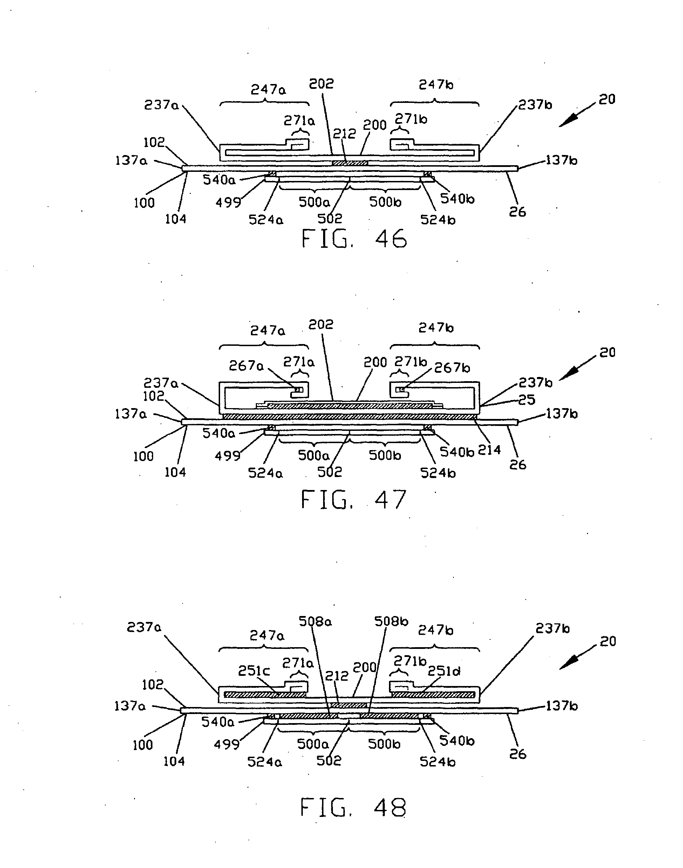

In the next example shown in **Figure 46** through **Figure 48** not showing embodiments of the present invention, a single discrete strip **499** is similarly exteriorly attached to the backsheet **26** at longitudinally extending laterally spaced attachment zones **540,** but these attachments are not releasable as in the preceding example. Instead, a pair of laterally abutted exteriorly disposed belt strips **500** is defined in the single discrete strip **499** by a single common frangible separation line **502** and two laterally outboard frangible separation lines **524,** at which the belt strips **500** can be detached for deployment. As in the previous examples, the common frangible separation line **502** corresponds to the first edges **520** of the belt strips **500** and the laterally outboard frangible separation lines **524** correspond to the second edges **522** of the respective belt strips **500.**

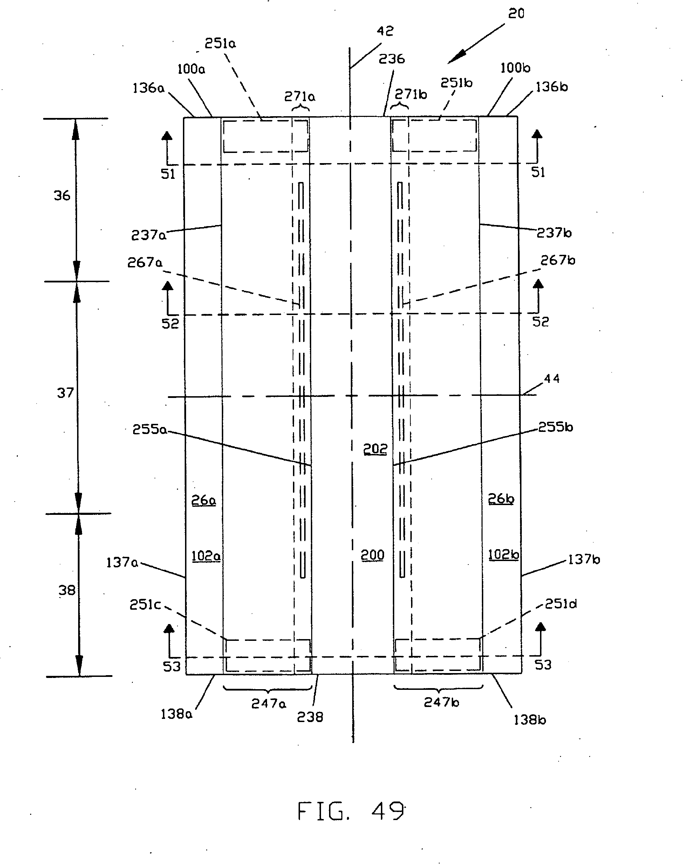

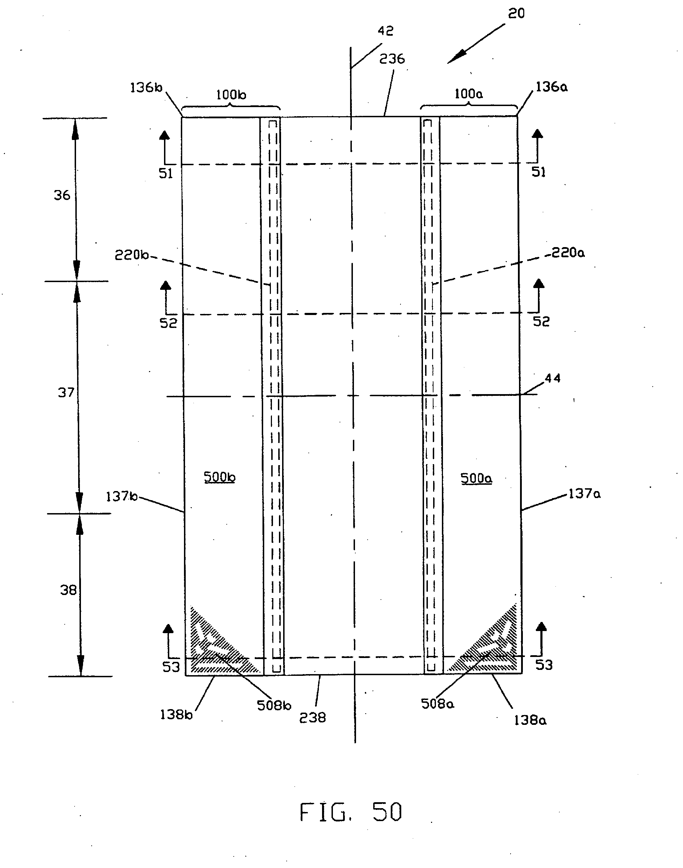

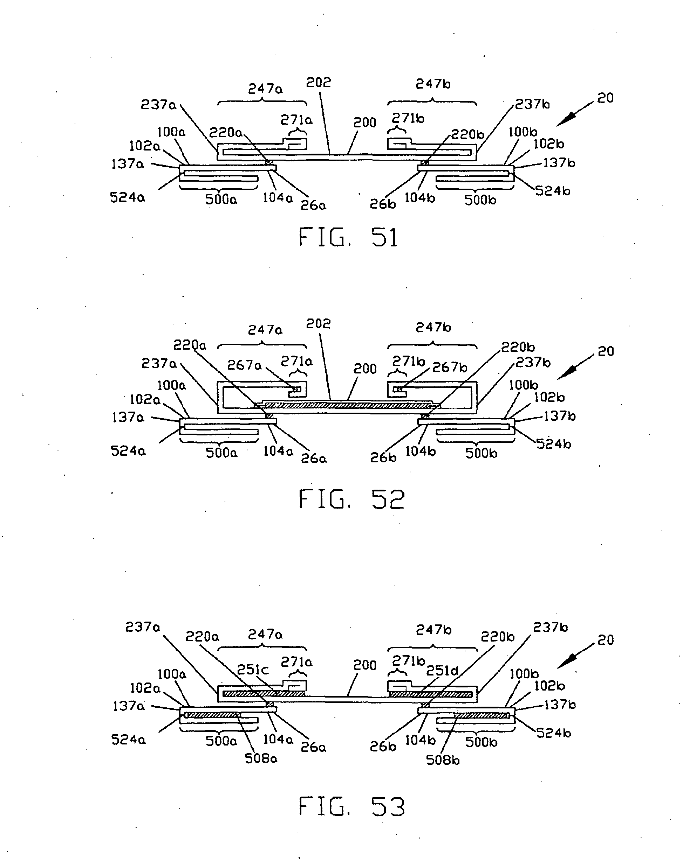

In the next example shown in **Figure 49** through **Figure 53****,** the backsheet **26** is folded laterally inward at or adjacent to the side edges **137** of the diaper **20** and the exteriorly disposed belt strips **500** are formed from the same layer as the backsheet **26,** as in the example shown in **Figure 38** through **Figure 43****.** However, in this example, the backsheet **26** is not laterally continuous. Instead, two laterally opposing longitudinally extending backsheet strips **26a** and **26b** are exteriorly attached to the absorbent assembly **200** in respective laterally opposing longitudinally extending attachment zones **220a** and **220b.**

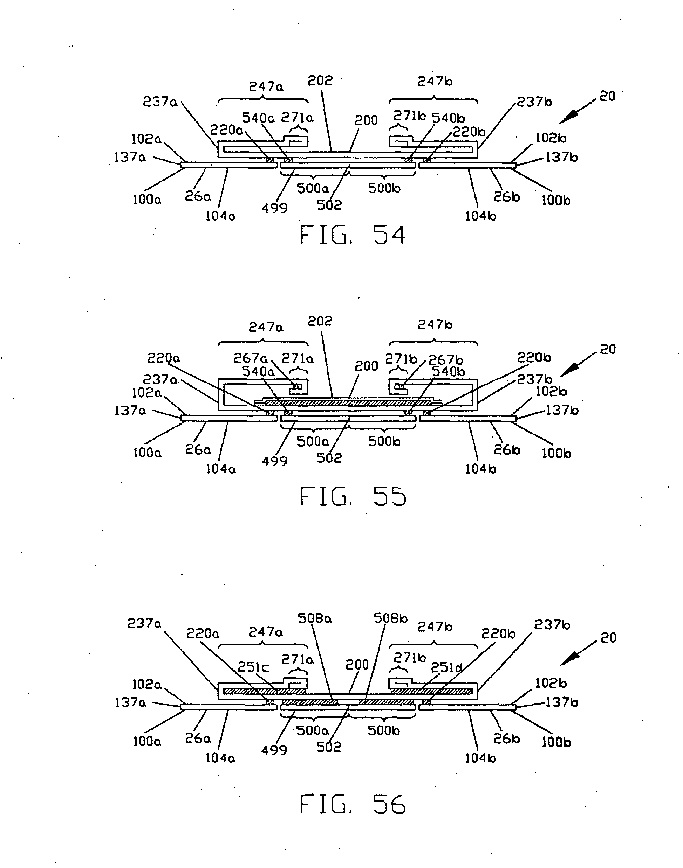

Two backsheet strips **26a** and **26b** are similarly exteriorly attached the absorbent assembly **200** in the example shown in **Figure 54** through **Figure 56** not showing embodiments of the present invention. However, in this example, a single discrete strip **499** is attached to the absorbent assembly **200** at longitudinally extending laterally spaced attachment zones **540.** A single common frangible separation line **502** located between the attachment zones **540** in this discrete strip **499** defines the two laterally abutted exteriorly disposed belt strips **500.** In this example, the belt strips **500** are releasably attached at the attachment zones **540** such that their deployment can be effected by detaching them there and at the frangible separation line **502.**

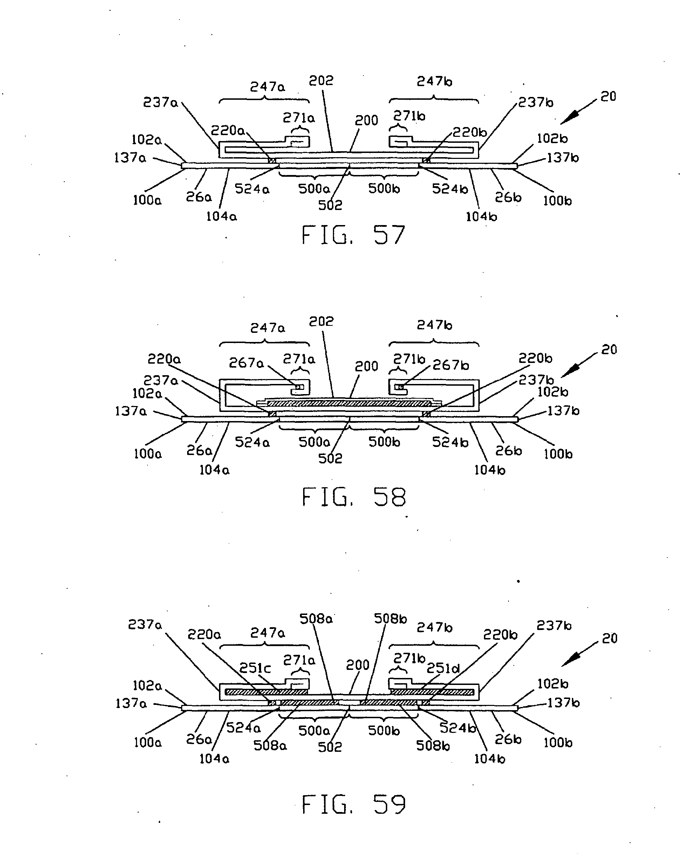

In the next example shown in **Figure 57** through **Figure 59****,** a pair of laterally abutted exteriorly disposed belt strips **500** is defined in the same layer as the backsheet strips **26a** and **26b** by a single common frangible separation line **502** and two laterally outboard frangible separation lines **524,** at which the belt strips **500** can be detached for deployment. As in the previous examples, the common frangible separation line **502** corresponds to the first edges **520** of the belt strips **500** and the laterally outboard frangible separation lines **524** correspond to the second edges **522** of the respective belt strips **500.**

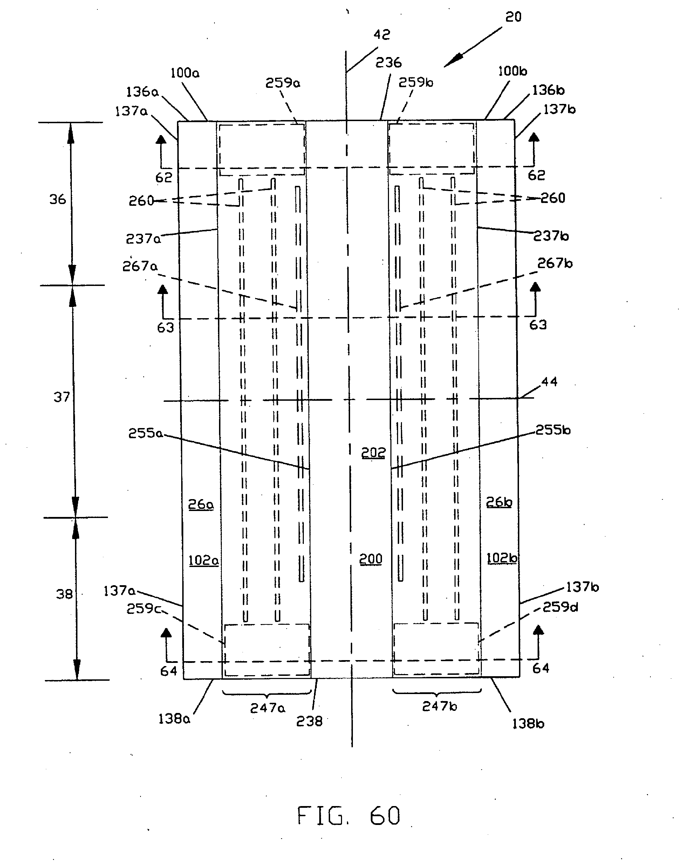

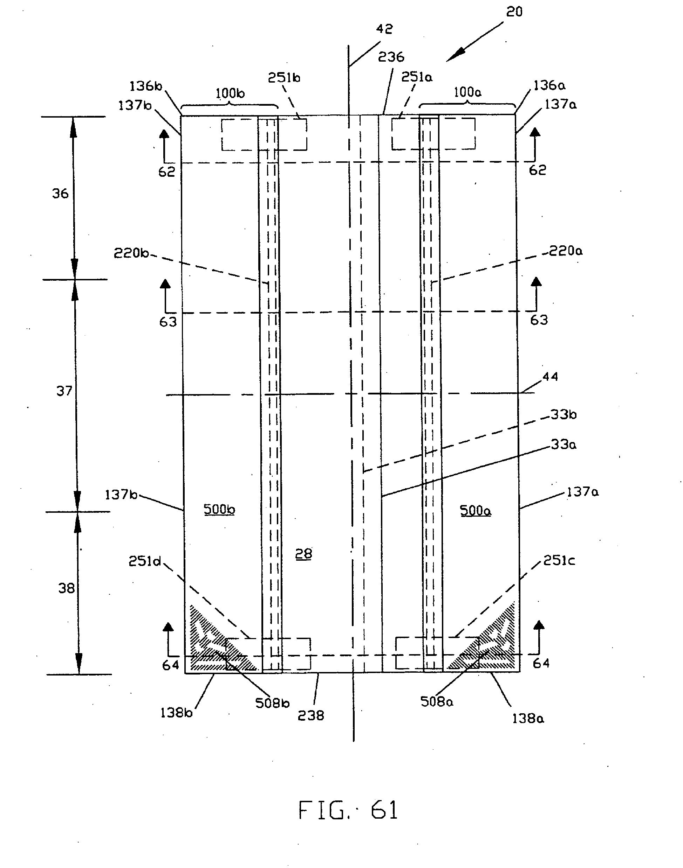

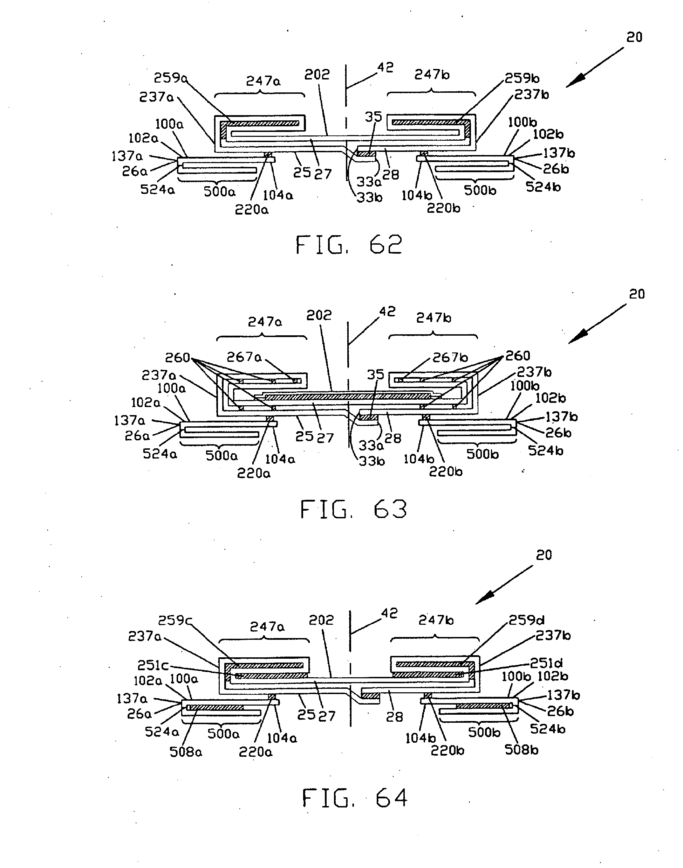

In the exemplary diaper **20** shown in **Figure 60** through **Figure 64****,** two laterally opposing longitudinally extending backsheet strips **26a** and **26b** arc again exteriorly attached to the absorbent assembly **200.** Each backsheet strip **26** is folded laterally inward and each exteriorly disposed belt strip **500** is formed from the same layer as the respective backsheet strip **26** by a laterally outboard frangible separation line **524.** In this example, a lower covering sheet **25** of the absorbent assembly **200** is doubled by folding and thereby includes a first layer **27** and a second layer **28** at least in the side flaps **247.** Doubling by folding is a particularly easy and cost-effective way of processing sheet materials in a manufacturing system, in part because it obviates the need to precisely align the edges of separate sheets when forming a doubled structure. In addition, doubling by folding makes it unnecessary to attach the doubled layers together, at least at the fold, although the layers can be attached together wherever desired for certain purposes, as explained below. In this embodiment, the lower covering sheet **25** is folded twice to form the two side flaps **247** and the layers **27** and **28** are overlapped and attached together adjacent to the original longitudinally extending edges **33** of the lower covering sheet **25** in the longitudinally extending attachment zone **35.**

The layers **27** and **28** of the doubled lower covering sheet **25** may remain unattached to each other and thus free to contact each other or separate from each other. Alternatively, the layers of the doubled lower covering sheet **25** may be attached together laterally continuously or intermittently. For example, the layers **27** and **28** may be attached together in laterally spaced attachment zones **260** extending longitudinally through the crotch region **37** and into the waist regions **36** and **38,** as shown in **Figure 60** through **Figure 64****.** Such longitudinally extending attachment together prevents the layers from separating and thereby presenting an undesirable baggy or blousy appearance around the legs of the wearer, as well as tending to stiffen the side flaps **247** slightly and thereby helping to ensure their proper fit against the body.

Alternatively, or in addition, the layers **27** and **28** of the doubled lower covering sheet **25** may be attached together in the waist regions **36** and **38** adjacent to the front and back edges **236** and **238** of the absorbent assembly **200,** for example in laterally extending attachment zones **259** as shown in the figures. This lateral attachment may be laterally intermittent or laterally continuous. When such laterally extending attachment is continuous, it prevents the layers from separating and thereby presenting an undesirable unfinished appearance at the waist edges, as well as forming a barrier serving to prevent the leakage of any liquid waste from between the layers at the front and/or back edge of the absorbent assembly.

Exemplary materials suitable for use in the doubled lower covering sheet **25** include breathable polyolefinic films, microporous or other breathable formed films, breathable monolithic films, and hydrophobic nonwovens. Suitable hydrophobic nonwovens include SM (spunbond meltblown), SMS (spunbond meltblown spunbond), and SMMS (spunbond meltblown meltblown spunbond) composites. The materials of the water vapor-permeable side sheets may be selected to balance overall product economics and function. For example, a relatively inexpensive nonwoven having a relatively low basis weight may provide the requisite level of water-impermeability when it is doubled, and its relatively low cost may offset the cost associated with the use of a greater amount of material than would be used in a single-layer lower covering sheet made of a relatively more expensive nonwoven material.

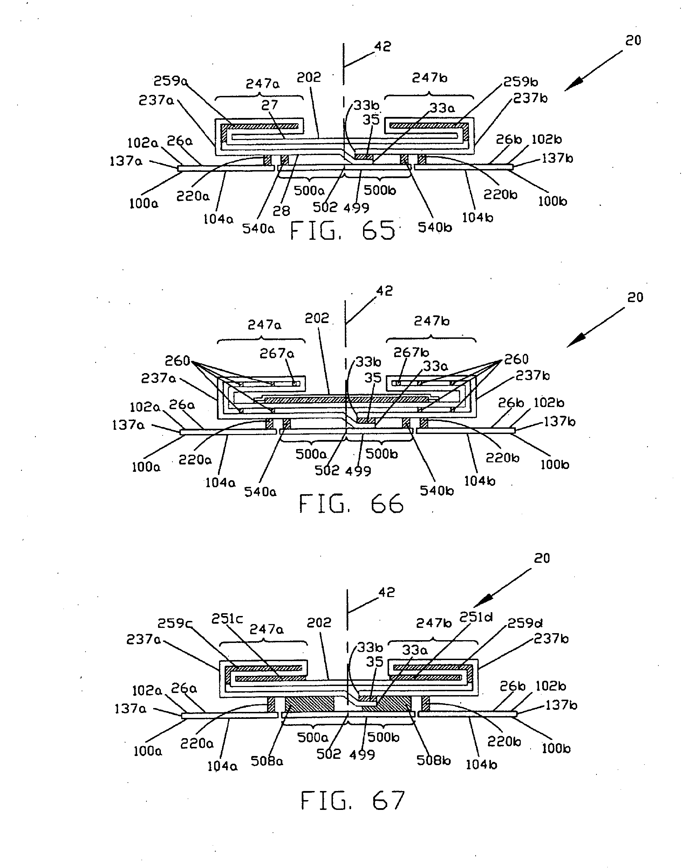

In the next example shown in **Figure 65** through **Figure 67** not showing embodiments of the present invention, two laterally opposing longitudinally extending backsheet strips **26a** and **26b** are again exteriorly attached to the absorbent assembly **200** and a lower covering sheet 25 of the absorbent assembly **200** is again doubled by folding. However, in this example, a single discrete strip **499** is attached to the absorbent assembly **200** at longitudinally extending laterally spaced attachment zones **540.** A single common frangible separation line **502** located between the attachment zones **540** in this discrete strip **499** defines the two laterally abutted exteriorly disposed belt strips **500.** In this example, the belt strips **500** are releasably attached at the attachment zones **540** such that their deployment can be effected by detaching them there and at the frangible separation line **502.**

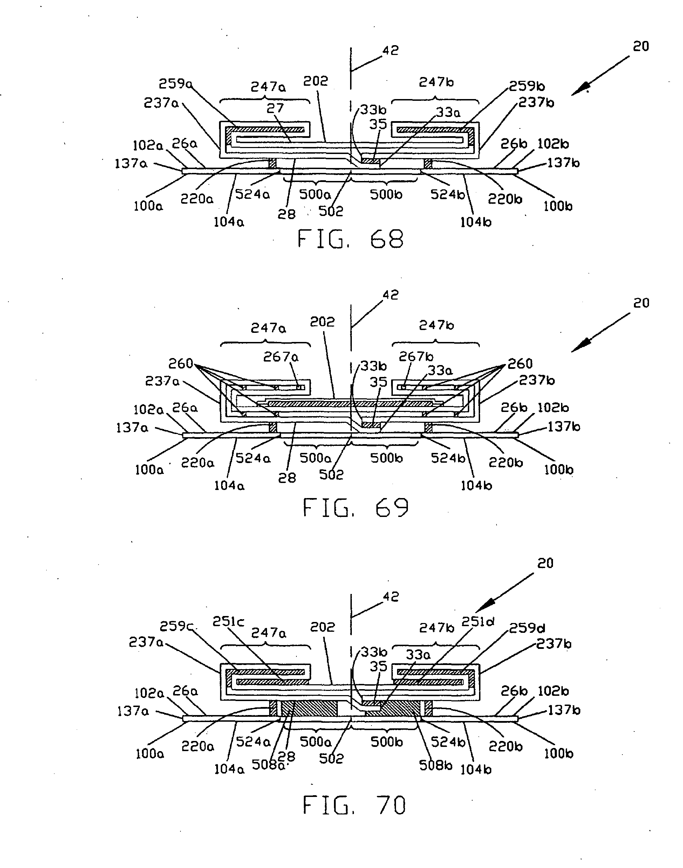

In the next example shown in **Figure 68** through **Figure 70****,** a lower covering sheet **25** of the absorbent assembly **200** is again doubled by folding. In this example, a pair of laterally abutted exteriorly disposed belt strips **500** is defined in the same layer as the backsheet strips **26a** and **26b** by a single common frangible separation line **502** and two laterally outboard frangible separation lines **524,** at which the belt strips **500** can be detached for deployment. As in the previous examples, the common frangible separation line **502** corresponds to the first edges **520** of the belt strips **500** and the laterally outboard frangible separation lines **524** correspond to the second edges **522** of the respective belt strips **500.**

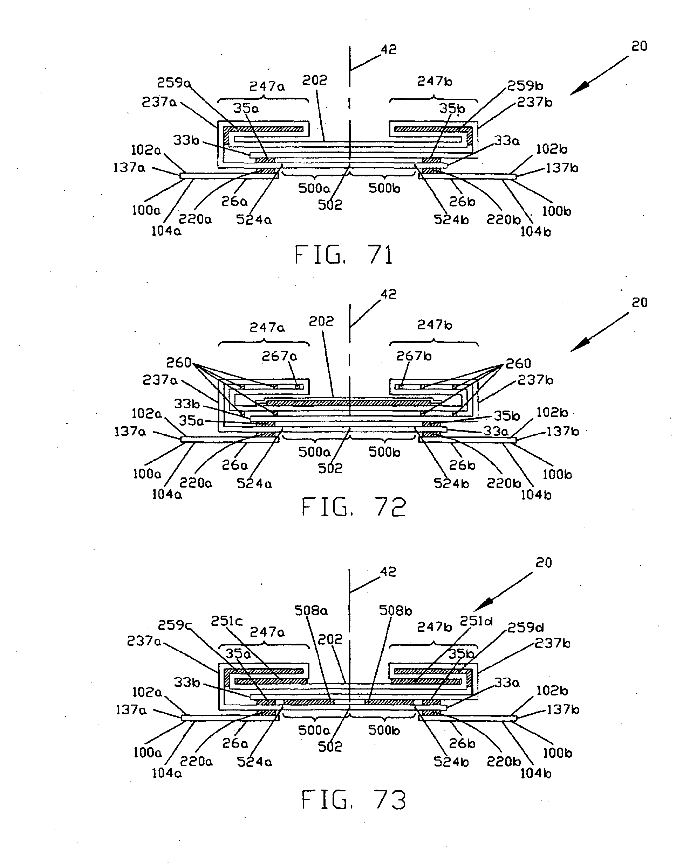

In the exemplary diaper **20** shown in **Figure 71** though **Figure 73****,** two laterally opposing longitudinally extending backsheet strips **26a** and **26b** are again exteriorly attached to the absorbent assembly **200** and a lower covering sheet **25** of the absorbent assembly **200** is again doubled by folding. However, in this example, the two layers **27** and **28** of the lower covering sheet **25** are attached together in two laterally opposing attachment zones **35a** and **35b,** instead of in a single attachment zone as in the preceding examples. In addition, a pair of laterally abutted exteriorly disposed belt strips **500** is defined in the same layer as the second layer **28** of the lower covering sheet **25** by a single common frangible separation line **502** and two laterally outboard frangible separation lines **524,** at which the belt strips **500** can be detached for deployment. Such a configuration may be used, for example, when it is desired to use the same material as the lower covering sheet **25** for the belt strips **500,** instead of the same material as the backsheet strips **100.**

The preceding examples are provided in order to convey to those of skill in the art that the deployable belt strips of the present invention can be provided in a variety of configurations. The above examples are not exhaustive, *i.e*., variations in addition to these are foreseen. For example, each of the mentioned layers may be formed of two or more members and thus may be laminates and/or composites of such members. As another example, each of the mentioned layers may be doubled by folding such that, for example, a belt strip **500** may be doubled and have one edge defined by a fold. The intent is to convey the concept of the present invention, *i.e*., a diaper incorporating deployable belt strips, while avoiding unnecessary length and complexity in this description. This voluntary characterization of the present invention is expressly not intended to constitute a surrender of any potential scope of any patentable claim(s).

## Claims

1. A disposable diaper (20) having longitudinally opposing front and back waist regions (36, 38) having waist edges (136, 138), laterally opposing side edges (137a, 137b) connecting the waist edges, a crotch region (37) between the waist regions, and comprising:
an absorbent assembly (200) having an interior surface (202) and comprising laterally opposing side flaps (247a, 247b) attached to the interior surface adjacent to the waist edges, each side flap having a longitudinally extending elastic gathering member (267a, 267b) attached adjacent to its proximal edge (255a, 255b);
a chassis (100) exteriorly attached to the absorbent assembly; and
at least one belt strip (500) having a fixed end portion (507) disposed in one of the waist regions, an opposing free end portion (516), a first edge (520) and a second edge (522), the first edge and the second edge connecting the end portions, the belt strip being attached in its fixed end portion (507) at both a laterally proximal end point (512) and a laterally distal end point (514) of a diagonal fold line (508) in an attachment zone (508)
the belt strip (500) being deployed for use by detaching the belt strip (500) except at its fixed end portion (507) and folding the belt strip (500) laterally outward at the diagonal fold line (506) such that the first edge extends laterally outward from the laterally proximal end point (512) of the diagonal fold line and the second edge extends laterally outward from the laterally distal end point (514) of the diagonal fold line,
wherein the belt strip (500) is formed contiguously with another structural element of the disposable diaper and is detachable from the other structural element along a frangible separation line (502, 524).

2. The disposable diaper of Claim 1 having only a single belt strip extending when deployed from the waist region where its fixed end portion is disposed to and laterally across the opposing waist region and further to the waist region where its fixed end portion is disposed and thereby connecting the waist regions at both of the side edges of the chassis.

3. The disposable diaper of Claim 1 having two laterally opposing belt strips.

4. The disposable diaper of Claim 3 wherein the belt strips are laterally spaced apart.

5. The disposable diaper of Claim 3 wherein the belt strips are laterally abutted.

6. The disposable diaper of any of Claims 3 through 5 wherein, after their deployment, the belt strips are tied together or, alternatively, are fastened together, exteriorly of the waist region opposing their fixed end portions.

7. The disposable diaper of any of Claims 1 and 3 through 5 having two laterally opposing belt strips (500c, 500d) having their fixed end portions disposed in the front waist region and two laterally opposing belt strips (500a, 500b) having their fixed end portions disposed in the back waist region.

8. The disposable diaper of Claim 7 wherein, after their deployment, two of the belt strips are tied together or, alternatively, are fastened together, adjacent to one of the side edges and the other two of the belt strips are tied together or, alternatively, are fastened together, adjacent to the opposing side edge.

## Patentansprüche

1. Wegwerfwindel (20) mit einander längsseitig entgegengesetzt liegenden vorderen und hinteren Taillenbereichen (36, 38), die Taillenränder (136, 138) aufweisen, seitlich entgegengesetzt liegenden Seitenrändern (137a, 137b), die die Taillenränder miteinander verbinden, einen Schrittbereich (37) zwischen den Taillenbereichen, und die Folgendes umfasst:
eine Absorptionsgruppe (200) mit einer Innenfläche (202) und die ferner seitlich entgegengesetzte Seitenklappen (247a, 247b) umfasst, die an der an die Taillenränder angrenzenden Innenfläche befestigt sind, wobei jede Seitenklappe ein längsseitig verlaufendes elastisches Raffungselement (267a, 267b) aufweist, das angrenzend an seinen proximalen Rand befestigt ist (255a, 255b);
einen Rahmen (100), der von außen an der Absorptionsgruppe befestigt ist; und
mindestens einen Gürtelstreifen (500) mit einem festen Endabschnitt (507), der in einem der Taillenbereiche angebracht ist, einen entgegengesetzten freien Endabschnitt (516), einen ersten Rand (520) und einen zweiten Rand (522), wobei der erste und der zweite Rand die Endabschnitte miteinander verbinden, wobei der Gürtelstreifen in seinem festen Endabschnitt (507) sowohl an einem seitlich proximalen Endpunkt (512) als auch einem seitlich distalen Endpunkt (514) einer diagonalen Faltlinie (506) in einem Befestigungsbereich (508) befestigt ist
wobei der Gürtelstreifen (500) durch Lösen des Gürtelstreifens (500) außer an seinem festen Endabschnitt (507) und durch Falten des Gürtelstreifens (500) seitlich nach außen an der diagonalen Faltlinie (506), so dass der erste Rand vom seitlich proximalen Endpunkt (512) der diagonalen Faltlinie seitlich nach außen verläuft und der zweite Rand vom distalen Endpunkt (514) der diagonalen Faltlinie seitlich nach auβen verläuft, zur Verwendung eingesetzt wird
wobei der Gürtelstreifen (500) angrenzend an ein anderes Strukturelement der Wegwerfwindel geformt ist und von dem anderen Strukturelement entlang einer reißbaren Trennlinie (502, 524) gelöst werden kann.

2. Wegwerfwindel nach Anspruch 1 mit nur einem einzigen Gürtelstreifen, der sich bei Verwendung vom Taillenbereich, an den sein fester Endabschnitt angebracht ist, bis zum und seitlich quer über den entgegengesetzten Taillenbereich erstreckt und weiter bis zum Taillenbereich, an dem sein fester Endabschnitt angebracht ist, wodurch die Taillenbereiche an beiden Seiten des Rahmens befestigt werden.

3. Wegwerfwindel nach Anspruch 1 mit zwei seitlich einander entgegengesetzten Gürtelstreifen.

4. Wegwerfwindel nach Anspruch 3, wobei die Gürtelstreifen seitlich voneinander mit Abstand angeordnet sind.

5. Wegwerfwindel nach Anspruch 3, wobei die Gürtelstreifen seitlich aneinanderstoßen.

6. Wegwerfwindel nach einem der Ansprüche 3 bis 5, wobei die Gürtelstreifen nach ihrer Verwendung außerhalb des Taillenbereichs, ihren festen Endabschnitten entgegengesetzt, zusammengebunden werden oder als Alternative dazu aneinander festgemacht werden.

7. Wegwerfwindel nach einem der Ansprüche 1 und 3 bis 5 mit zwei seitlich entgegengesetzten Gürtelstreifen (500c, 500d), deren feste Endabschnitte im vorderen Taillenbereich angeordnet sind, und zwei seitlich entgegengesetzten Gürtelstreifen (500a, 500b), deren feste Endabschnitte im hinteren Taillenbereich angeordnet sind.

8. Wegwerfwindel aus Anspruch 7, wobei zwei der Gürtelstreifen nach ihrer Verwendung an einen der Seitenränder angrenzend zusammengebunden oder als Alternative dazu aneinander festgemacht werden und die anderen beiden Gürtelstreifen an den entgegengesetzten Seitenrand angrenzend zusammengebunden oder als Alternative dazu aneinander festgemacht werden.

## Revendications

1. Couche jetable (20) ayant des régions de ceinture avant et arrière (36, 38) longitudinalement opposées ayant des bords de ceinture (136, 138), des bords latéraux latéralement opposés (137a, 137b) reliant les bords de ceinture, une région d'entrejambe (37) entre les régions de ceinture, et comprenant :
un assemblage absorbant (200) ayant une surface intérieure (202) et comprenant des rabats latéraux latéralement opposés (247a, 247b) fixés à la surface intérieure adjacente aux bords de ceinture, chaque rabat latéral ayant un élément de rassemblement élastique s'étendant longitudinalement (267a, 267b) fixé adjacent à son bord proximal (255a, 255b) ;
un châssis (100) fixé extérieurement à l'assemblage absorbant ; et
au moins une bande de ceinture (500) ayant une partie terminale fixe (507), disposée dans une des régions de ceinture, une partie d'extrémité libre opposée (516), un premier bord (520) et un deuxième bord (522), le premier bord et le deuxième bord reliant les parties terminales, la bande de ceinture étant fixée dans la partie terminale fixe (507) à la fois à un point terminal latéralement proximal (512) et à un point terminal latéralement distal (514) d'une ligne de pliure diagonale (506) dans une zone d'attachement (508)
la bande de ceinture (500) étant déployée pour une utilisation en détachant la bande de ceinture (500) sauf à sa partie terminale fixe (507) et en pliant la bande de ceinture (500) latéralement vers l'extérieur à la ligne de pliure diagonale (506) de telle sorte que le premier bord s'étend latéralement vers l'extérieur à partir du point terminal latéralement proximal (512) de la ligne de pliure diagonale et le deuxième bord s'étend latéralement vers l'extérieur à partir du point terminal distal (514) de la ligne de pliure diagonale, dans laquelle la bande de ceinture (500) est formée de façon contiguë avec un autre élément structural de la couche jetable et est détachable de l'autre élément structural le long d'une ligne de séparation frangible (502, 524).

2. Couche jetable selon la revendication 1, ayant uniquement une seule bande de ceinture s'étendant lorsqu'on la déploie à partir de la région de ceinture où sa partie terminale fixe est disposée et latéralement à travers la région de ceinture opposée et encore jusqu'à la région de ceinture où sa partie terminale fixe est disposé et en reliant de ce fait les régions de ceinture à l'un et l'autre des bords latéraux du châssis.

3. Couche jetable selon la revendication 1 ayant deux bandes de ceinture latéralement opposées.

4. Couche jetable selon la revendication 3, dans laquelle les bandes de ceinture sont espacées latéralement.

5. Couche jetable selon la revendication 3, dans laquelle les bandes de ceinture sont aboutées latéralement.

6. Couche jetable selon l'une quelconque des revendications 3 à 5, dans laquelle, après leur déploiement, les bandes de ceinture sont nouées ensemble ou, en variante, sont attachées ensemble, à l'extérieur de la région de ceinture opposée à leurs parties terminales fixes.

7. Couche jetable selon l'une quelconque des revendications 1 et 3 à 5 ayant deux bandes de ceinture latéralement opposées (500c, 500d) ayant leurs parties terminales fixes disposées dans la région de ceinture avant et deux bandes de ceinture latéralement opposées (500a, 500b) ayant leurs parties terminales fixes disposées dans la région de ceinture arrière.

8. Couche jetable selon la revendication 7, dans laquelle, après leur déploiement, deux des bandes de ceinture sont nouées ensemble ou, en variante, sont attachées ensemble, adjacentes à un des bords latéraux et les deux autres des bandes de ceinture sont nouées ensemble ou, en variante, sont attachées ensemble, adjacentes au bord latéral opposé.
